# EUROPÄISCHE PATENTANMELDUNG

(11) **EP 3 321 375 A1**
(43) Veröffentlichungstag der Anmeldung: **16.05.2018**
(21) Anmeldenummer: 16198800.1
(22) Anmeldetag: 15.11.2016
(51) Int. Cl.: C12Q 1/68

(54) **VERFAHREN ZUM NACHWEIS VON DELETIONEN, DUPLIKATIONEN ODER INVERSIONEN DER GLEICHEN KRITISCHEN REGION**

(71) Anmelder: ZytoVision GmbH, 27572 Bremerhaven (DE)
(72) Erfinder: Dr. Marggraf-Rogalla, Piere, 28816 Stuhr (DE); Dr. Hauke, Sven, 28203 Bremen (DE)
(74) Vertreter: Meissner Bolte Partnerschaft mbB

(57) **Zusammenfassung**

Die Erfindung betrifft eine Zusammensetzung zum Nachweis struktureller Chromosomenaberrationen in einer Probe, umfassend drei Sonden, wobei a) die erste Sonde ein erstes Nukleinsäurefragment umfasst, das mit einem ersten Marker markiert ist, b) die zweite Sonde ein zweites Nukleinsäurefragment umfasst, das mit einem zweiten Marker markiert ist, und c) die dritte Sonde ein drittes Nukleinsäurefragment umfasst, das mit einem dritten Marker markiert ist, wobei der erste, der zweite und der dritte Marker verschiedene Marker sind, wobei das erste, das zweite und das dritte Nukleinsäurefragment jeweils spezifisch an einen komplementären Abschnitt auf einem Chromosom binden und die drei Abschnitte auf demselben Chromosom liegen, und wobei die drei Sonden im spezifisch an das Chromosom gebundenen Zustand Abstände voneinander aufweisen, durch welche die drei Marker räumlich getrennt voneinander nachgewiesen werden können und, sofern in und zwischen den Abschnitten keine Aberration vorliegt, in der räumlichen Reihenfolge erster - zweiter - dritter Marker nachgewiesen werden können. Darüber hinaus betrifft die Erfindung auch Verfahren zum Nachweis struktureller Chromosomenaberrationen in einer Probe, Schritte umfassend, bei denen man a) eine biologische Probe, die Chromosomen umfasst, mit einer erfindungsgemäßen Zusammensetzung unter Bedingungen in Kontakt bringt, die eine Hybridisierung der Sonden mit komplementären Abschnitten auf den Chromosomen erlauben; und b) die räumliche Reihenfolge der drei Marker nachweist.

## Beschreibung

### Hintergrund

Die vorliegende Erfindung betrifft eine Zusammensetzung sowie Verfahren zum Nachweis struktureller Chromosomenaberrationen in einer Probe.

Vielen genetischen Erkrankungen, wie Tumorerkrankungen und Mikrodeletions- und Mikroduplikationssyndromen, liegen strukturelle Chromosomenmutationen wie Translokationen, Inversionen und Deletionen zugrunde. Der Nachweis dieser Veränderungen als prädiktiver, prognostischer oder differentialdiagnostischer Marker geschieht in der Regel durch In-Situ-Hybridisierungen (ISH) unter Verwendung von Fluoreszenz-markierten (Fluoreszente ISH (FISH)) Nukleinsäurefragmenten, sogenannten Sonden, oder Hapten-markierten Sonden, welche anschließend mit Antikörpern detektiert und durch Farbreaktionen lichtmikroskopisch (Hellfeld ISH (BrISH) - dazu zählen u.a. auch Chromogene ISH (CISH) und Silber ISH (SISH)) - sichtbar gemacht werden.

Ein Vorteil der FISH ist, dass ohne Mehraufwand in der Durchführung der Methode multiple genomische Bereiche simultan, aber voneinander deutlich unterscheidbar, nachgewiesen werden können. Hierzu werden Nukleinsäurefragmente, die spezifisch in unterschiedlichen genomischen Bereichen binden, mit verschiedenen Fluoreszenzfarbstoffen markiert, d. h. gekoppelt. Die Fluoreszenzfarbstoffe unterscheiden sich in ihrem Absorptions- und/oder Emissionsspektrum voneinander. Werden solche mehrfarbigen Sonden z. B. an Metaphase-Chromosomen-Präparaten oder an Interphase-Zellkern-Präparaten verwendet, so können die einzelnen Farben durch Verwendung spezifischer Mikroskop-Filter-Sets, die genau definierte Wellenlängenbereiche des Lichts zur Anregung der Farbstoffe auf das Präparat leiten sowie genau definierte Wellenlängenbereiche des von den Farbstoffen emittierten Lichts zum Auswerter durchlassen, getrennt voneinander dargestellt werden (sog. Single-Bandpass-Filterset) oder aber es werden die unterschiedlichen Fluoreszenzsignale mehrerer Fragmente (bei zwei unterschiedlichen Fluoreszenzfarbstoffen spricht man hierbei von einem Dual-Bandpass-Filterset) gleichzeitig dargestellt.

Der simultanen Darstellung sind jedoch deutliche Grenzen gesetzt, da die Absorptions-und Emissionsbereiche der Farbstoffe häufig so nah beieinander liegen, dass sie durch die Mikroskop-Filter-Sets nicht voneinander getrennt werden können. Außerdem führen zwei sich überlagernde Signale unterschiedlicher Farben zur Wahrnehmung von Mischfarben (z. B. ergeben rot (oder orange) und grün dabei gelb), die sich nicht unterscheiden lassen von gleichfarbigen Signalen, die das Resultat eines anderen Fluorochroms sind und nicht durch Überlappung entstanden. Aus diesen Gründen werden in der Routine-FISH simultan zumeist nur zwei Farben (orange/rot und grün) bzw. drei Farben (orange/rot und grün simultan mit einer blauen Kerngegenfärbung (DAPI)) betrachtet. In etwa die gleichen Einschränkungen, die für die FISH darstellbar sind, gelten für die BrISH. Hier ist der Stand der Technik die Verwendung von zwei Haptenen, zumeist ausgewählt aus der Gruppe Biotin, Dinitrophenyl (DNP) und Digoxigenin, und zwei antikörpergekoppelten Enzymen, zumeist alkalische Phosphatase und Peroxidase (Carbone et al., 2008; Hopman et al., 1997; Laakso et al., 2006; Mayr et al., 2009; Tanner et al., 2000).

Die genannten Einschränkungen bei der simultanen Darstellung haben einen maßgeblichen Einfluss auf die Kompositionen der Sonden für den Nachweis von Translokationen und Inversionen, aber auch von Deletionen und Duplikationen. Hier gibt es prinzipiell zwei maßgebliche Techniken und zugrunde liegende Sondenkompositionen die Translokationen und Inversionen betreffend sowie eine maßgebliche Technik und zugrunde liegende Sondenkomposition die Deletionen betreffend: Das Prinzip des Entstehens von Fusionssignalen (sog. Dual-Color-Dual-Fusion-Ansätze) (WO 02/093130, Dewald et al., 1998; Dewald et al., 2000; Wan et al., 2003) bzw. des sich Auftrennens von Fusionssignalen (sog. Dual-Color-Break-Apart- bzw. Dual-Color-Split-Ansätze) (van der Burg et al., 1999; Boomer et al., 2001; van der Burg et al., 2004) sowie des Verlustes oder Zugewinns von Signalen im Vergleich zu einer Kontrollsonde (sog. Genspezifische Sonden-Ansätze).

In der nachfolgenden Darstellung dieser Prinzipien und abgeleiteten Signalmuster ist zu beachten, dass eine normale Zelle in der Regel diploid ist, d. h. jedes Allel zweifach vorliegt. Da von Aberrationen in der Regel jeweils nur eines der beiden Allele betroffen ist, ist neben dem aberranten Signal üblicherweise auch noch das normale Signal des nicht von der Aberration betroffenen Allels sichtbar. Zum besseren Verständnis wird nachfolgend das Signalmuster des normalen Signals nicht immer explizit beschrieben.

Bei Dual-Color-Dual-Fusion-Ansätzen wird der Bereich des Bruchpunkts 1 proximal und distal von Nukleinsäurefragmenten, die mit der gleichen Farbe (z. B. orange) markiert sind, flankiert, der Bereich des Bruchpunktes 2, d. h. des reziproken Translokationspartners, wird proximal und distal von Nukleinsäurefragmenten, die mit einer zweiten Farbe (z. B. grün) markiert sind, flankiert. Die normale Situation, d. h. ohne chromosomale Brüche im Bereich der beiden Translokationspartner, ist hierbei durch ein grünes und ein räumlich getrenntes oranges Signal charakterisiert.

Im Falle einer reziproken Translokation kommt es innerhalb der Bruchpunkte beider Translokationspartner zu Brüchen und der proximale Bereich des einen Translokationspartners fusioniert mit dem distalen Bereich des anderen Partners und *vice versa.* Es entstehen somit zwei grün/orange Signal-Paare, auch Fusionssignale genannt, da sich die unterschiedlich farbigen Signale oftmals überlappen, so dass ein mischfarbenes Signal sichtbar ist. Der Nachteil dieser Sonden-Techniken ist, dass die Fusionssignale nur entstehen, wenn die Bruchpunkte beider Translokationspartner im Bereich der jeweiligen markierten Nukleinsäurefragmente liegen. Bei Translokationen, die nur einen der beiden Partner betreffen, entstehen keine Fusionssignale. Es kommt hierbei lediglich zur Entstehung eines zusätzlichen Signals mit der Farbe der Sonde, die charakteristisch für den durch die Translokation betroffenen Partner ist. D. h. es entsteht z. B. ein zusätzliches grünes Signal wenn der Bruchpunkt der Translokation in dem Bereich lag, der von den mit grünem Fluorochrom markierten Nukleinsäuren überdeckt wurde. Solche Zusatzsignale können jedoch häufig durch andere Zellkerne überdeckt werden, dicht bei einem der anderen gleichfarbigen Signale liegen und somit nicht als Zusatzsignal wahrgenommen werden, oder durch Gewebsschnittartefakte verloren gehen. In diesem Fall kann es zur Falschdiagnose kommen, da eine nur den einen Translokationspartner betreffende Translokation nicht wahrgenommen wird.

Bei Dual-Color-Break-Apart-Ansätzen wird der Bereich eines Bruchpunktes proximal und distal von unterschiedlich markierten oder farbig-markierten Nukleinsäurefragmenten flankiert (z. B. distal orange, proximal grün). Die normale Situation, d. h. ohne chromosomalen Bruch dieses Bereiches, wird dabei durch ein Fusionssignal charakterisiert. In einer aberranten Situation, d. h. wenn es zu einem chromosomalen Bruch zwischen den Sondenfragmenten kommt, trennen sich die Signale räumlich voneinander. Der Unterschied zwischen der normalen Situation und der aberranten Situation ist also durch den Abstand der unterschiedlich farbigen Signale gekennzeichnet. Der Nachteil dieser Sonden-Kompositionen ist, dass Aussagen über den beteiligten Translokationspartner nicht möglich sind. Außerdem kann es, bedingt z. B. durch die Sichtachse mit der der Betrachter auf den Interphasezellkern sieht, dazu kommen, dass zwei räumlich getrennte Signale, die in Bezug auf die Sichtachse übereinander liegen, weiterhin als Fusionssignal wahrgenommen werden.

Bei genspezifischen Sonden-Ansätzen wird der Bereich, der von einer chromosomalen Deletion oder Duplikation (auch Amplifikationen, die im Folgenden nicht adressiert werden) betroffen sein kann, und ein in der Regel weit entfernter Kontrollbereich, bevorzugt die Zentromerregion des entsprechenden Chromosoms, mit unterschiedlich markierten oder farbig-markierten Nukleinsäurefragmenten adressiert (z. B. Deletions-oder Duplikationsbereich orange, Kontrollbereich grün). Die normale Situation, d. h. ohne Deletion oder Duplikation, wird dabei durch ein oranges Signal charakterisiert. Wenn es zu einer chromosomalen Deletion kommt, geht das orange Signal verloren. Im Fall einer chromosomalen Duplikation tritt ein weiteres oranges Signal auf.

Neben den o. g. Zweifarb-Anwendungen werden in FISH-Analysen auch Dreifarb-, Vierfarb- und Fünffarbsonden verwendet. Dabei werden neben den deutlich stärkeren Standard-Fluoreszenzfarben orange/rot und grün die weiteren, zur Verfügung stehenden schwächeren Farben, z. B. gold oder rot oder blau verwendet. Daher werden in Routineanwendungen beispielweise Vierfarb-FISH-Sonden nur zum Nachweis der Verluste ganzer Chromosomen verwendet (EP 1035215 B1, WO 2007/028031, EP 0549709 B1), weil hier zumeist bei den Markierungen der Sonden auf repetitive Sequenzen zur Verstärkung der Farbintensitäten z. B. von blau und gold zurückgegriffen werden kann.

Betreffend BrISH unter Verwendung von mehr als zwei Farben beschreiben Hopman et al. (1997) die Durchführung einer chromogenen Dreifach-*In*-*situ*-Hybridisierung, die allgemein auf den Nachweis von drei repetitiven chromosomalen Bereichen abzielt. Nach dem derzeitigen Stand der Technik werden Translokationen mittels BrISH nur unter Verwendung von zwei Haptenen und somit zwei Farbstoffen nachgewiesen.

Inversionen, die in einer Parentalgeneration bzw. Zellen einer Parentalgeneration entstehen, können dazu führen, dass die Zellen einer Filialgeneration von Deletionen oder auch Duplikationen in der zuvor invertierten Region betroffen sind. Dabei können a) bei Tumorerkrankungen Deletionen oder Duplikationen in Tumorzellen (Filialgeneration) im Vergleich zu den Inversionen der Zellen des Umgebungs- und Ursprungsgewebes (Parentalgeneration) oder b) auch bei erblichen Tumoren Deletionen oder Duplikationen in Zellen von Kindern (Filialgeneration) im Vergleich zu den Inversionen der Zellen der Eltern (Parentalgeneration) oder c) insbesondere auch bei den sog. Mikrodeletions- und Mikroduplikationssyndromen Deletionen oder Duplikationen in fetalen, embryonalen, kindlichen oder adulten Zellen des Kindes (Filialgeneration) im Vergleich zu den Inversionen der Zellen der Eltern (Parentalgeneration) sichtbar und diagnostisch von entscheidender Bedeutung sein.

Davon abweichend gibt es auch Fälle, bei denen die Deletionen oder Duplikationen selbst von den zuvor genannten Zellen einer Parentalgeneration auf Zellen einer Filialgeneration vererbt werden.

Betreffend die Mikrodeletions- und Mikroduplikationssyndrome, die primär von Deletionen/Inversionen/Duplikationen kleiner als 20 Mb betroffen sind, gibt es keine geeigneten Sonden, die entsprechende Inversionen in den elterlichen Zellen nachweisen können.

Alle derzeit verfügbaren Methoden und Sondenkompositionen für BrISH und FISH im Zusammenhang mit Inversionen und Deletionen oder Duplikationen, insbesondere bei Mikrodeletions- und Mikroduplikationssyndromen, weisen Mängel auf. So gibt es keine Sondenkompositionen, die in jedem Fall die sichere Erkennung einer Inversion von Zellen einer Parentalgeneration und gleichzeitig einer Deletion oder Duplikation von Zellen einer Filialgeneration erlauben.

Aufgabe der Erfindung ist es daher, eine Sondenkomposition bereitzustellen, die einen sicheren Nachweis struktureller Chromosomenaberrationen ermöglicht, wobei es sich bei den strukturellen Chromosomenaberrationen sowohl um Inversionen als auch Deletionen oder Duplikationen der gleichen kritischen genomischen Region handeln kann und die strukturellen Chromosomenaberrationen insbesondere Mikrodeletions- und Mikroduplikationssyndrome sein können.

### Beschreibung

Gelöst wird die Aufgabe durch eine Zusammensetzung zum Nachweis struktureller Chromosomenaberrationen in einer Probe, welche die kennzeichnenden Merkmale des Anspruchs 1 aufweist, sowie durch ein Verfahren zur Verwendung der Zusammensetzung, welches die kennzeichnenden Merkmale des Anspruchs 13 aufweist. Die Erfindung betrifft eine Zusammensetzung umfassend drei unterschiedlich markierte Sonden, die im spezifisch an ein Chromosom gebundenen Zustand räumlich getrennt voneinander, d. h. nicht als Fusionssignale, und in der räumlichen Reihenfolge erster-zweiter- dritter Marker nachgewiesen werden können.

Die Erfindung betrifft insbesondere eine Zusammensetzung zum Nachweis struktureller Chromosomenaberrationen in einer Probe, umfassend drei Sonden, wobei
a) die erste Sonde ein erstes Nukleinsäurefragment umfasst, das mit einem ersten Marker markiert ist,
b) die zweite Sonde ein zweites Nukleinsäurefragment umfasst, das mit einem zweiten Marker markiert ist, und
c) die dritte Sonde ein drittes Nukleinsäurefragment umfasst, das mit einem dritten Marker markiert ist,
wobei der erste, der zweite und der dritte Marker verschiedene Marker sind,
wobei das erste, das zweite und das dritte Nukleinsäurefragment jeweils spezifisch an einen komplementären Abschnitt auf einem Chromosom binden und die drei Abschnitte auf demselben Chromosom liegen, und
wobei die drei Sonden im spezifisch an das Chromosom gebundenen Zustand Abstände voneinander aufweisen, durch welche die drei Marker räumlich getrennt voneinander nachgewiesen werden können und, sofern in und zwischen den Abschnitten keine Aberration vorliegt, in der räumlichen Reihenfolge erster - zweiter - dritter Marker nachgewiesen werden können.

Es wurde überraschenderweise festgestellt, dass bei der Auswahl eines geeigneten Abstands zwischen den drei Sonden und einer geeigneten Platzierung der Sonden um potentielle Bruchstellen herum, die Sonden räumlich getrennt voneinander, aber in der räumlichen Reihenfolge, in der sie an die Primärsequenz des Chromosoms binden, nachgewiesen werden können. So ermöglicht die Erfindung äußerst präzise Angaben zu Chromosomenaberrationen in dem markierten Bereich, insbesondere auch Aberrationen sehr kleiner Chromosomenabschnitte, z. B. Mikrodeletionen, Mikroduplikationen und Mikroinversionen. Solche MMSs (Microdeletion and microduplication syndromes) sind beispielsweise in Weise et al., 2012 (J Histochem Cytochem, 60:346) beschrieben.

Mittels der erfindungsgemäßen Zusammensetzung können, mit anderen Worten, mehrere unterschiedliche Chromosomen- oder DNS-Bereiche in einer Zelle nachgewiesen (d. h. detektiert) werden. Die Zusammensetzung ist zum Nachweis struktureller Chromosomenaberrationen geeignet. Die Zusammensetzung ist insbesondere geeignet, sowohl Inversionen als auch Deletionen oder Duplikationen der gleichen kritischen genomischen Region(en) eindeutig nachzuweisen. Die Zusammensetzung ist besonders zum Nachweis von Mikrodeletionen, Mikroduplikationen und/oder Mikroinversionen geeignet. Eine "strukturelle Chromosomenabberration" im Sinne der Erfindung ist eine Veränderung des Aufbaus eines Chromosoms, wie z. B. eine Translokation, eine Inversion und/oder eine Deletion.

Das erfindungsgemäße Verfahren erlaubt gegenüber den o. g. bekannten Verfahren und Kompositionen erstmals einen eindeutigen und/oder überhaupt erstmals möglichen Nachweis von Inversionen und/oder Deletionen und/oder Duplikationen einer gleichen kritischen genomischen Region.

Das erfindungsgemäße Verfahren basiert gegenüber den o. g. bekannten Verfahren und Kompositionen erstmals darauf, dass - insbesondere bei Mikrodeletions- und Mikroduplikationssyndromen - nicht die Anzahl von Signalen für den Nachweis von Inversionen und/oder Deletionen und/oder Duplikationen einer gleichen kritischen genomischen Region entscheidend ist oder primär entscheidend ist, sondern insbesondere auch die Reihenfolge bzw. Anordnung der Signale. Die Sondenkomposition der vorliegenden Erfindung kombiniert auch die zuvor genannten Ansätze für Inversionen und Deletionen oder Duplikationen, d.h. Dual-Color-Break-Apart-Ansatz, Dual-Color-Fusion-Ansatz und Genspezifischer Sonden-Ansatz. Allerdings kommt es betreffend die Inversionen nicht auf Break-Apart- oder Fusions-Ereignisse an, sondern auf die geänderten Signalmuster bzw. Signalreihenfolgen (z. B. Signale A-B-C in der normalen, 'gesunden' Situation, die sich zu beispielsweise B-A-C in der invertierten Situation verändern). Insbesondere die Deletionen und Duplikationen betreffend, wird eine dritte Sonde, die in relativ geringem Abstand außerhalb der kritischen Chromosomenregion liegt, (Sonde C bzw. Marker C) als Ersatz für die in Standardverfahren weiter entfernten, zumeist zentromerspezifischen, Kontroll-Sonden bzw. -Marker verwendet.

Die erfindungsgemäße Zusammensetzung wird zum *in vitro* Nachweis struktureller Chromosomenaberrationen in einer Probe verwendet. Die Probe ist eine biologische Probe, z. B. eine Probe, die einem Menschen oder einem Tier entnommen wurde. Vorzugsweise ist die Probe eine menschliche Probe. Die Probe umfasst Chromosomen, vorzugsweise Chromosomen in der Metaphase und/oder Interphase. "Chromosom in der Metaphase bzw. Interphase" ist erfindungsgemäß gleichbedeutend mit "Chromosomen einer Zelle, die sich in der Metaphase bzw. Interphase befindet". In einer Ausführungsform umfasst die Probe Chromosomen in der Metaphase. Alternativ oder zusätzlich kann die Probe aber auch Chromosomen in der Interphase umfassen. Es ist bevorzugt, dass die Probe sowohl Chromosomen in der Metaphase als auch Chromosomen in der Interphase umfasst. Dazu kann die Probe z. B. Zellen, Zellkerne und/oder Teile davon umfassen. Vorzugsweise ist die Probe eine histologische Probe, vorzugsweise eine histologische Gewebeprobe, z. B. ein histologischer Schnitt. Alternativ kann die Probe aber auch eine Suspension oder ein Präparat einzelner Zellen sein.

Die erfindungsgemäße Zusammensetzung umfasst drei Sonden. Dies bedeutet, dass die Zusammensetzung mindestens drei Sonden umfasst, sie aber auch mehr Sonden enthalten kann, z. B. vier, fünf, sechs oder mehr Sonden. In einer Ausführungsform umfasst die Zusammensetzung genau drei, d. h. nicht mehr als drei, Sonden. "Sonden" wird hierbei gleichbedeutend mit "Sondenarten" verwendet. Dies bedeutet, dass die Zusammensetzung (mindestens) drei unterschiedliche Sondenarten umfasst, wobei von jeder einzelnen Sondenart eine Vielzahl von Sondenmolekülen in der Zusammensetzung enthalten ist. Unterschiedliche Sondenarten bzw. Sonden im Sinne der Erfindung umfassen ein Nukleinsäurefragment, das sich in seiner Primärsequenz von den Nukleinsäurefragmenten anderer Sondenarten bzw. Sonden in der Zusammensetzung unterscheidet und einen Marker, der sich von den Markern anderer Sondenarten bzw. Sonden unterscheidet.

Die Zusammensetzung umfasst drei Sonden, wobei a) die erste Sonde ein erstes Nukleinsäurefragment umfasst, das mit einem ersten Marker markiert ist, b) die zweite Sonde ein zweites Nukleinsäurefragment umfasst, das mit einem zweiten Marker markiert ist, und c) die dritte Sonde ein drittes Nukleinsäurefragment umfasst, das mit einem dritten Marker markiert ist. Eine "Sonde" im Sinne der Erfindung ist, mit anderen Worten, ein direkt (kovalent) oder indirekt mit einem Marker (Label) markiertes Nukleinsäurefragment. Die Sonde kann aus dem Nukleinsäurefragment und dem Marker bestehen oder weitere Komponenten umfassen. Beispielsweise kann eine Sonde im Sinne der Erfindung chemische Modifikationen, insbesondere Modifikationen an den 5' und/oder 3' Enden der Sonde umfassen. Solche Modifikationen können z. B. zur Stabilisierung der Sonde dienen. Es ist auch nicht ausgeschlossen, dass die Sonde neben dem ersten, zweiten bzw. dritten Marker einen weiteren Marker umfasst. Dieser wird sich jedoch von den in den anderen beiden Sonden verwendeten Markern unterscheiden und getrennt von diesen nachweisbar sein.

Die nachweisbaren Sonden bzw. Markersignale werden hierin vereinzelt zur Vereinfachung auch als Sonden/Signale "A" (erste Sonde), "B" (zweite Sonde) und "C" (dritte Sonde) bezeichnet.

Die Nukleinsäurefragmente sind vorzugsweise Poly- oder Oligonukleotide. Oligonukleotide sind Nukleinsäurefragmente mit einer Länge von 2 bis 100, vorzugsweise 10 bis 30 Nukleotideinheiten. Polynukleotide sind Nukleinsäurefragmente mit einer Länge von mehr als 30 Nukleotideinheiten, vorzugsweise mehr als 50 Nukleotideinheiten, stärker bevorzugt mehr als 100 Nukleotideinheiten.

Die Nukleinsäurefragmente weisen vorzugsweise eine Länge von 50 kb (kilobasen) bis 1000 kb auf, stärker bevorzugt von 80 kb bis 500 kb, am stärksten bevorzugt von 100 kb bis 300 kb.

Die Nukleinsäurefragmente sind einzelsträngig oder doppelsträngig. Doppelsträngige Nukleinsäurefragmente werden vor der Hybridisierung mit einer Zielsequenz zu Einzelsträngen denaturiert. Entsprechende Verfahren zur Denaturierung und zur Hybridisierung mit einer Zielsequenz sind Fachleuten auf dem Gebiet der *In-Situ-*Hybridisierung bekannt.

Die Nukleinsäurefragmente sind vorzugsweise DNS (Desoxyribonukleinsäure) - Fragmente. Die Nukleinsäurefragmente können aber auch modifizierte Nukleinsäurefragmente sein, wie z. B. Peptid-Nukleinsäurefragmente (PNS-Fragmente) oder *'locked nucleic acid*'- Fragmente (LNS-Fragmente). Dem Fachmann sind weitere geeignete Nukleinsäuren und Modifikationen bekannt.

Die Nukleinsäurefragmente sind jeweils mit einem Marker markiert (gelabelt). Der erste, der zweite und der dritte Marker sind voneinander verschiedene (d. h. unterschiedliche) Marker. Dies bedeutet, dass die drei Marker getrennt voneinander nachweisbar sind. Der erste, zweite und dritte Marker können zum Beispiel drei unterschiedliche Fluoreszenzfarbstoffe sein (die z. B. mittels FISH Verfahren nachweisbar sind) oder drei unterschiedliche Reportermoleküle sein (die z. B. mittels BrISH Verfahren nachweisbar sind). In einer bevorzugten Ausführungsform sind der erste, zweite und dritte Marker drei unterschiedliche Fluoreszenzmoleküle.

Allgemein können die Marker ausgewählt sein aus der Gruppe bestehend aus Farbstoffen, Farbstoffsubstraten, Radioisotopen, Spin-Markern, Enzymen, Haptenen, Quantum Dots, Beads, Aminohexylen und Pyren. Geeignete Farbstoffe sind z. B. Chemilumineszenzfarbstoffe (wie z. B. Acridinium) und Fluoreszenzfarbstoffe. Die Fluoreszenzfarbstoffe sind z. B. ausgewählt aus der Gruppe bestehend aus Fluorescein, Fluorescein-Derivat, 5(6)-Carboxyfluorescein, Coumarin, Coumarin-Derivat, Rhodamin, Rhodamin-Derivat, Tetramethylrhodamin, Lissamin, Texas Red, AMCA, TRITC, IR Farbstoff, Alexa-Farbstoff, Dyomics-Farbstoff, Phycoerythrine, Cascade Blue, Oregon Green 488, Pacific Blue und Rhodamine Green. Geeignete Enzyme sind z. B. Alkalische Phosphatase, Meerrettich-Peroxidase, Sojabohnen-Peroxidase und beta-Galactosidase. Die Haptene können z. B. ausgewählt sein aus der Gruppe bestehend aus Dioxigenin, Biotin, 5(6)-Carboxyfluorescein, Rhodamin, Bromdeoxyuridin, Acetylaminofluoren, Trinitrophenol, Trinitrophenol - Derivaten, Estradiol und DNP.

Der erste, zweite und dritte Marker sind bevorzugt Fluoreszenzfarbstoffe für die Emissionsbereiche Grün, Orange/Rot bzw. Blau. In einer alternativen Ausführungsform sind der erste, zweite und dritte Marker Fluoreszenzfarbstoffe für die Emissionsbereiche Grün, Rot und Gold. In diesen Ausführungsformen sind die Marker bevorzugt kovalent (d. h. direkt) mit den Nukleinsäurefragmenten verbunden, d. h. direkt in die jeweilige Sonde eingebaut. Alternativ können die Fluoreszenzfarbstoffe aber auch z. B. über Komplexe indirekt mit den Nukleinsäurefragmenten verbunden sein. Solche und alternative Markierungsmöglichkeiten mit Fluoreszenzfarbstoffen sind Fachleuten bekannt.

Weiter bevorzugt sind die Marker für die Verwendung als FISH-Marker geeignet. Erfindungsgemäß spielt es keine Rolle, welcher der drei Marker ein Fluoreszenzfarbstoff für welchen der oben genannten Emissionsbereiche ist, d. h. die räumliche Reihenfolge der nachgewiesenen Emissionsbereiche spielt keine Rolle für die Effizienz des Nachweisverfahrens.

In einer weiteren bevorzugten Ausführungsform sind der erste, zweite und dritte Marker Biotin, Digoxigenin und DNP. Auch hierbei spielt es keine Rolle, welcher der drei Marker welches der genannten Haptene ist, d. h. die räumliche Reihenfolge der drei Haptene spielt keine Rolle für die Effizienz des Nachweisverfahrens. Biotin, Digoxigenin und DNP binden an mit Antikörper-gekoppelte alkalische Phosphatase, Antikörper-gekoppelte Peroxidase und Antikörper-gekoppelte beta-Galactosidase und können auf diese Weise nachgewiesen werden.

Die Nukleinsäurefragmente sind jeweils mit einem Marker markiert. Dies bedeutet, dass mindestens ein Markermolekül direkt, d. h. kovalent, oder indirekt, d. h. nicht kovalent, mit dem Nukleinsäuremolekül verbunden ist. Es können erfindungsgemäß auch zwei oder mehr Markermoleküle mit einem einzelnen Nukleinsäuremolekül verbunden sein, wobei die Markermoleküle identisch sind. Hierdurch kann das jeweilige Signal verstärkt werden.

Das erste, das zweite und das dritte Nukleinsäurefragment binden jeweils spezifisch an einen komplementären Abschnitt auf einem Chromosom.

Die Bindung der Nukleinsäurefragmente erfolgt, wie Fachleuten bekannt, über die Ausbildung von Basenpaaren zwischen dem Nukleinsäurefragment und dem jeweiligen komplementären Abschnitt auf einem Chromosom. Fachleuten sind entsprechende Verfahren zum Design und zur Herstellung von komplementären Nukleinsäurefragmenten für bestimmte Zielsequenzen bekannt.

Wie üblich meint "spezifische" Bindung, dass das jeweilige Nukleinsäurefragment im Wesentlichen (d. h. in nachweisbarem Ausmaß) nur an den komplementären Abschnitt im Chromosom bindet und im Wesentlichen nicht (d. h. nicht in nachweisbaren Ausmaß) an andere DNS Abschnitte in der Probe.

Die drei (komplementären) Abschnitte liegen auf demselben Chromosom. Dies bedeutet, dass die drei Abschnitte auf demselben Chromosom liegen, sofern die Abschnitte nicht von einer Aberration (insbesondere einer Deletion) betroffen sind. Sind die Abschnitte von einer Aberration betroffen, z. B. von einer Deletion, so versteht es sich, dass unter Umständen einzelne der drei komplementären Abschnitte nicht mehr auf dem Chromosom liegen. Selbstverständlich können andere, mit der vorliegenden Zusammensetzung detektierbare Aberrationen, wie z. B. eine Inversion oder Duplikation, dazu führen, dass alle Abschnitte weiterhin auf demselben Chromosom liegen.

Das menschliche Genom weist zwischen 23 (Frau) und 24 (Mann) verschiedene Chromosomen auf. Die komplementären Abschnitte können somit z. B. auf dem Chromosom 1, 2, 3, 4, 5, 6, 7, 8, 9, 10, 11, 12, 13, 14, 15, 16, 17, 18, 19, 20, 21, 22, X oder Y liegen. Vorzugsweise liegen die Abschnitte auf einem Chromosom, das ausgewählt ist aus der Gruppe bestehend aus Chromosomen 1, 6 und 7.

In einer Ausführungsform binden das erste und zweite Nukleinsäurefragment beide in demselben Chromosomenbereich (einer "kritischen" Region), wobei der Chromosomenbereich ausgewählt ist aus den Chromosomenbereichen, die bei Weise et al. (2012) beschrieben werden. In einer weiteren Ausführungsform binden das erste und zweite Nukleinsäurefragment beide in demselben Chromosomenbereich (einer "kritischen" Region), wobei der Chromosomenbereich ausgewählt ist aus der Gruppe bestehend aus 15q11-q13 (Angelman-Syndrom), 5p15.2-p15.3 (Cri-du-chat-Syndrom), 22q11.2 (DiGeorge-Syndrom), 17p13.3 (Miller-Dieker-Syndrom), 15q11-q13 (Prader-Willi-Syndrom), 22q11.2 (Shprintzen-Syndrom), 17p11.2 (Smith-Magenis-Syndrom), 7q11.23 (Williams-Beuren-Syndrom), 4p16.3 (Wolf-Hirschhorn-Syndrom), 1q21.1 (Mikrodeletion 1q21.1), 1q21.1 (Mikroduplikation 1q21.1), 1q41q42 (Mikrodeletion 1q41q42), 2p15p16.1 (Mikrodeletion 2p15p16.1), 3q29 (Mikrodeletion 3q29), 7q11.23 (Mikroduplikation 7q11.23), 9q22.3 (Mikrodeletion 9q22.3), 12q14 (Mikrodeletion 12q14), 14q11.2 (Mikrodeletion 14q11.2), 15q13.3 (Mikrodeletion 15q13.3), 15q24 (Mikrodeletion 15q24), 16p11.2 (Mikrodeletion/Duplikation 16p11.2), 16p11.2p12.2 (Mikrodeletion 16p11.2p12.2), 16p13.1 (Mikrodeletion 16p13.1), 16p13.1 (Mikroduplikation 16p13.1), 17p11.2 (Potocki-Lupski Syndrom), 17p11.2 (Mikroduplikation 17p11.2), 17q21.31 (Mikrodeletion 17q21.31), 19q13.11 (Mikrodeletion 19q13.11), 22q11.2 (Distale Mikrodeletion 22q11.2), Xq28 (Mikroduplikation Xq28), 1 p32.1-p31.1 (Mikrodeletionen und -duplikation 1p32-p31), 7q32.2-q34 (Mikrodeletionen 7q33) und 6q22.33-q23.3 (Mikrodeletionen 6q22.33). In einer besonders bevorzugten Ausführungsform binden das erste und zweite Nukleinsäurefragment beide in demselben Chromosomenbereich, wobei der Chromosomenbereich ausgewählt ist aus der Gruppe bestehend aus 1 p32.1-p31.1 (Mikrodeletionen und -duplikation 1p32-p31), 7q32.2-q34 (Mikrodeletionen 7q33) und 6q22.33-q23.3 (Mikrodeletionen 6q22.33).

Das dritte Nukleinsäurefragment bindet vorzugsweise außerhalb des Chromosomenbereichs bzw. der kritischen Region, in der das erste und das zweite Nukleinsäurefragment binden.

Die drei Sonden weisen im spezifisch an das Chromosom, vorzugsweise an das Chromosom in der Metaphase, gebundenen Zustand Abstände voneinander auf, durch welche die drei Marker räumlich getrennt voneinander nachgewiesen werden können und, sofern in und zwischen den Abschnitten keine Aberration vorliegt, in der räumlichen Reihenfolge erster - zweiter - dritter Marker nachgewiesen werden können. Wie bereits erwähnt, basiert die Anwendung der erfindungsgemäßen Zusammensetzung und der damit verbundene Nachweis der Marker, erstmals nicht nur auf der Trennung und/oder Fusion bestimmter Signale, sondern auf der räumlichen Reihenfolge der Markersignale. Es wurde im Rahmen der Erfindung überraschenderweise festgestellt, dass die hierin angegebenen Abstände sowohl für den räumlich getrennten Nachweis in der Metaphase als auch in der Interphase geeignet sind. Somit weisen die drei Sonden in einer bevorzugten Ausführungsform im spezifisch an das Chromosom in der Metaphase und in der Interphase gebundenen Zustand Abstände voneinander auf, durch welche die drei Marker räumlich getrennt voneinander nachgewiesen werden können und, sofern in und zwischen den Abschnitten keine Aberration vorliegt, in der räumlichen Reihenfolge erster - zweiter - dritter Marker nachgewiesen werden können.

Erfindungsgemäß entspricht die nachgewiesene räumliche Reihenfolge der Marker, sofern in und zwischen den Abschnitten keine Aberration vorliegt, der Reihenfolge in der die jeweiligen korrespondierenden Nukleinsäurefragmente an die Primärsequenz des Chromosoms binden. Dies bedeutet, dass die Nukleinsäurefragmente in der (räumlichen) Reihenfolge erstes - zweites - drittes Nukleinsäurefragment an das Chromosom binden, sofern in und zwischen den Abschnitten keine Aberration vorliegt.

Räumlich getrennte Signale im Sinne der Erfindung sind Signale, die als distinkte Einzelsignale nachgewiesen werden können, insbesondere als distinkte Einzelsignale in Chromosomen in der Metaphase. Die Marker sind somit als voneinander getrennte Einzelsignale nachweisbar, insbesondere als voneinander getrennte Einzelsignale in Chromosomen in der Metaphase. Die Marker weisen einen Abstand auf, der groß genug ist, um im Wesentlichen keine Fusionssignale zu erzeugen, insbesondere um keine Fusionssignale in Chromosomen in der Metaphase zu erzeugen.

Es versteht sich, dass Fusionssignale in vereinzelten Zellen auch bei weit voneinander entfernten Markern dadurch entstehen können, dass die Sichtachse ungünstig gewählt ist. Die Aussage, dass die Marker erfindungsgemäß einen Abstand aufweisen, der groß genug ist, um im Wesentlichen keine Fusionssignale zu erzeugen, bezieht sich somit auf die Mehrzahl der untersuchten Zellen. Mit anderen Worten, weisen die drei Sonden Abstände voneinander auf, durch die in mindestens 50 % aller Fälle (d. h. aller Zellen) die drei Marker räumlich getrennt voneinander, d. h. nicht als Fusionssignale, nachgewiesen werden können, vorzugsweise in mindestens 70 % aller Fälle, stärker bevorzugt in mindestens 90 % aller Fälle. Ein "Fall" ist der Nachweis in einer Zelle bzw. der Nachweis eines Chromosoms.

Ob ein Fusionssignal entsteht oder nicht hängt auch von der für den Nachweis verwendeten Auflösung ab. So kann bei Verwendung einer weniger hohen Auflösung (z. B. einer 200x Vergrößerung) ein Fusionssignal erkennbar sein, dass sich in einer höheren Auflösung (z. B. einer 630x Vergrößerung) in distinkte Einzelsignale aufspaltet. Bevorzugt weisen die drei Sonden Abstände voneinander auf, durch welche die drei Marker in einer Vergrößerung von 630x räumlich getrennt voneinander nachgewiesen werden können. Fachleute auf dem Gebiet sind in der Lage geeignete Vergrößerungen für einen optimalen Nachweis zu identifizieren.

Ob ein Fusionssignal entsteht, hängt auch vom Kondensationsgrad der Chromosomen bzw. des Chromatins ab. So kann bei der Betrachtung von Metaphasen-Chromosomen ein Fusionssignal erkennbar sein, das sich bei der Betrachtung der Interphasen in distinkte Einzelsignale aufspaltet. Es ist daher besonders bevorzugt, dass das Chromosom im Sinne der Erfindung ein Chromosom in der Metaphase ist, d. h. ein Metaphase-Chromosom. Es ist jedoch mit der erfindungsgemäßen Zusammensetzung möglich, strukturelle Chromosomenaberrationen gleichermaßen an Metaphase- und Interphase-Chromosomen nachzuweisen.

Die Nukleinsäurefragmente weisen je nach zu untersuchendem Chromosomen-Kondensationsgrad im spezifisch an das Chromosom gebundenen Zustand Abstände auf, durch welche die drei Marker räumlich getrennt voneinander nachgewiesen werden können und, sofern in und zwischen den Abschnitten keine Aberration vorliegt, in der der räumlichen Reihenfolge erster - zweiter - dritter Marker nachgewiesen werden können. In einer Ausführungsform weisen die Nukleinsäurefragmente im spezifisch an ein Chromosom in der Metaphase gebundenen Zustand Abstände auf, durch welche die drei Marker räumlich getrennt voneinander nachgewiesen werden können und, sofern in und zwischen den Abschnitten keine Aberration vorliegt, in der der räumlichen Reihenfolge erster - zweiter - dritter Marker nachgewiesen werden können. In einer alternativen Ausführungsform weisen die Nukleinsäurefragmente im spezifisch an ein Chromosom in der Interphase gebundenen Zustand Abstände auf, durch welche die drei Marker räumlich getrennt voneinander nachgewiesen werden können und, sofern in und zwischen den Abschnitten keine Aberration vorliegt, in der der räumlichen Reihenfolge erster - zweiterdritter Marker nachgewiesen werden können. In einer besonders bevorzugten Ausführungsform weisen die Nukleinsäurefragmente sowohl im spezifisch an ein Chromosom in der Metaphase als auch an ein Chromosom in der Interphase gebundenen Zustand Abstände auf, durch welche die drei Marker räumlich getrennt voneinander nachgewiesen werden können und, sofern in und zwischen den Abschnitten keine Aberration vorliegt, in der der räumlichen Reihenfolge erster - zweiter - dritter Marker nachgewiesen werden können. Der Begriff "Abstand" bezeichnet hierbei den Abstand zwischen dem Ende des ersten und dem Anfang des zweiten Nukleinsäurefragments und zwischen dem Ende des zweiten und dem Anfang des dritten Nukleinsäurefragments (jeweils im spezifisch an das Chromosom gebundenen Zustand). So kann der Begriff "Abstand" z. B. den Abstand zwischen dem 3'-Ende des ersten und dem 5'-Ende des zweiten Nukleinsäurefragments und zwischen dem 3'-Ende des zweiten und dem 5'-Ende des dritten Nukleinsäurefragments bezeichnen.

Es wurde insbesondere gefunden, dass diese räumliche Signaltrennung erreicht wird, wenn die Nukleinsäurefragmente einen Abstand von mindestens 50 kb aufweisen, z. B. mindestens 500 kb (0,5 Mb), vorzugsweise von mindestens 1000 kb (1 Mb), stärker bevorzugt von mindestens 2000 kb (2Mb). Somit sind in bestimmten Ausführungsformen im spezifisch gebundenen Zustand das 3' Ende des ersten Nukleinsäurefragments vom 5' Ende des zweiten Nukleinsäurefragments und das 3' Ende des zweiten Nukleinsäurefragments vom 5' Ende des dritten Nukleinsäurefragments jeweils mehr als 50 kb voneinander entfernt, z. B. mindestens 500 kb (0,5 Mb), vorzugsweise 1000 kb (1 Mb), stärker bevorzugt mindestens 2000 kb (2 Mb).

Die Abstände zwischen den drei Sonden sind ferner derart gewählt, dass, sofern in und zwischen den Abschnitten keine (mit der Sondenkombination nachweisbare) Aberration vorliegt, d. h. im 'gesunden' Zustand, die Marker in der räumlichen Reihenfolge erster - zweiter - dritter Marker nachgewiesen werden können (d. h. in der gleichen Reihenfolge, in der die jeweils zugehörigen Nukleinsäurefragmente an die Primärsequenz des Chromosoms binden). Der Begriff "Reihenfolge", wie hierin verwendet, umfasst sowohl die räumliche Reihenfolge der drei Marker als auch die Anzahl der nachweisbaren Markersignale (d. h. die Anzahl des ersten, des zweiten und des dritten Markers) pro Chromosom. Erfindungsgemäß wird somit mittels der erfindungsgemäßen Zusammensetzung und des erfindungsgemäßen Verfahrens nicht nur die räumliche Reihenfolge der einzelnen Marker sondern auch die Anzahl der Markersignale in einer Zelle oder an einem Chromosom nachgewiesen.

Es wurde gefunden, dass ein Nachweis der Marker in der Reihenfolge, in der die Sonden an dem Chromosom angeordnet sind, bei Wahl entsprechend geringer Abstände zwischen den Sonden möglich ist. Somit betrifft die Erfindung insbesondere eine Zusammensetzung, wobei im spezifisch gebundenen Zustand der Abstand zwischen dem ersten und dem zweiten Nukleinsäurefragment und der Abstand zwischen dem zweiten und dem dritten Nukleinsäurefragment jeweils weniger als 20 Mb ist, vorzugsweise weniger als 15 Mb, stärker bevorzugt weniger als 12 Mb, noch stärker bevorzugt weniger als 9 Mb, am stärksten bevorzugt weniger als 7 Mb. So kann das 3' Ende des ersten Nukleinsäurefragments (Sonde A) vom 5' Ende des zweiten Nukleinsäurefragments (Sonde B) und das 3' Ende des zweiten Nukleinsäurefragments (Sonde B) vom 5' Ende des dritten Nukleinsäurefragments (Sonde C) jeweils weniger als 20 Mb entfernt sein, vorzugsweise weniger als 15 Mb, stärker bevorzugt weniger als 12 Mb, noch stärker bevorzugt weniger als 9 Mb, am stärksten bevorzugt weniger als 7 Mb. In einer besonders bevorzugten Ausführungsform betragen diese Entfernungen bzw. Anstände weniger als 7 Mb. Mit anderen Worten ist der Abstand zwischen dem ersten und dem zweiten Nukleinsäurefragment, z. B. der Abstand zwischen dem 3' Ende des ersten Nukleinsäurefragments (Sonde A) und dem 5' Ende des zweiten Nukleinsäurefragments (Sonde B) und der Abstand zwischen dem 3' Ende des zweiten Nukleinsäurefragments (Sonde B) und dem 5' Ende des dritten Nukleinsäurefragments (Sonde C), von 50 kb (50.000 bp) bis 20 Mb, vorzugsweise von 50 kb bis 15 Mb, stärker bevorzugt von 50 kb bis 9 Mb, noch stärker bevorzugt von 50 kb bis 7 Mb. In besonders bevorzugten Ausführungsformen ist der Abstand zwischen dem ersten und dem zweiten Nukleinsäurefragment und der Abstand zwischen dem zweiten und dem dritten Nukleinsäurefragment, z. B. der Abstand zwischen dem 3' Ende des ersten Nukleinsäurefragments (Sonde A) und dem 5' Ende des zweiten Nukleinsäurefragments (Sonde B) und der Abstand zwischen dem 3' Ende des zweiten Nukleinsäurefragments (Sonde B) und dem 5' Ende des dritten Nukleinsäurefragments (Sonde C), von 0,5 Mb bis 20 Mb, vorzugsweise von 1 Mb bis 15 Mb, besonders bevorzugt von 2 Mb bis 8 Mb.

Die erfindungsgemäßen Sonden sind somit geeignet, eine Inversion, Deletion oder Duplikation eines genomischen Abschnitts nachzuweisen, die weniger als 20 Mb umfasst.

Der Abstand zwischen den Nukleinsäurefragmenten wird erfindungsgemäß jeweils zwischen dem Ende eines der gebundenen Nukleinsäurefragmente und dem Anfang des nächsten Nukleinsäurefragments angegeben, z. B. zwischen dem 3' Ende des einen und dem 5' Ende des nächsten Nukleinsäurefragments.

Vorzugsweise ist im spezifisch gebundenen Zustand insbesondere der Abstand zwischen dem ersten und dem zweiten Nukleinsäurefragment, z. B. der Abstand zwischen dem 3' Ende des ersten Nukleinsäurefragments (Sonde A) und dem 5' Ende des zweiten Nukleinsäurefragments (Sonde B), weniger als 10 Mb, stärker bevorzugt weniger als 8 Mb, am stärksten bevorzugt weniger als 6 Mb. In einer besonders bevorzugten Ausführungsform beträgt diese Entfernung bzw. dieser Abstand weniger als 6 Mb. Mit anderen Worten ist dieser Abstand vorzugsweise von 50 kb bis 10 Mb, stärker bevorzugt von 1 Mb bis 7 Mb.

Weiter vorzugsweise ist im spezifisch gebundenen Zustand insbesondere der Abstand zwischen dem zweiten Nukleinsäurefragment und dem dritten Nukleinsäurefragment, z. B. der Abstand zwischen dem 3' Ende des zweiten Nukleinsäurefragments (Sonde B) und dem 5' Ende des dritten Nukleinsäurefragments (Sonde C), von 20 Mb bis 1 Mb, bevorzugt von 18 Mb bis 2 Mb, besonders bevorzugt von 15 Mb bis 3 Mb und noch bevorzugter von 10 Mb bis 3,5 Mb. Mit anderen Worten beträgt diese Entfernung bzw. dieser Abstand vorzugsweise weniger als 7 Mb.

Um die erfindungsgemäße Erkenntnis optimal für den Nachweis bestimmter bekannter Chromosomenaberrationen zu nutzen, werden die komplementären Abschnitte auf dem Chromosom vorzugsweise derart gewählt, dass eine oder mehrere bekannte Bruchstellen zwischen den komplementären Abschnitten liegen. Vorzugsweise liegen dabei die komplementären Abschnitte zu dem ersten und dem zweiten Nukleinsäurefragment (Sonden A und B) in einer kritischen Region, wobei eine kritische Region ein Chromosomenabschnitt ist, von dem bekannt ist, dass es zu verschiedenen Aberrationsereignissen in diesem Abschnitt kommen kann, z. B. zu Deletionen, Inversionen und/oder Duplikationen. Vorzugsweise liegt der komplementäre Abschnitt zu dem dritten Nukleinsäurefragment (Sonde C) außerhalb der kritischen Region, so dass insbesondere eine bekannte potentielle Bruchstelle zwischen dem zweiten und dem dritten Nukleinsäurefragment (Sonden B und C) liegt (wobei diese Bruchstelle vorzugsweise die kritische Region begrenzt). In einer Ausführungsform liegt jeweils eine bekannte, potentielle Bruchstelle zwischen dem ersten und dem zweiten Nukleinsäurefragment (zwischen Sonden A und B) und zwischen dem zweiten und dem dritten Nukleinsäurefragment (zwischen Sonden B und C).

Das von der dritten Sonde umfasste Nukleinsäurefragment bindet bevorzugt unmittelbar außerhalb, z. B. angrenzend an, die kritische Region. Der Abstand zwischen der Sonde und der kritischen Region kann z. B. weniger als 5 Mb, vorzugsweise weniger als 3 Mb, besonders bevorzugt weniger als 1 Mb betragen.

Die Marker können - sofern in und zwischen den komplementären Abschnitten keine Aberration vorliegt - in der räumlichen Reihenfolge erster - zweiter - dritter Marker nachgewiesen werden (d. h. in der Reihenfolge Sonde/Sondensignal A - B - C). Dies bedeutet, dass sich der komplementäre Abschnitt in einer ersten möglichen Ausführungsform zu dem zweiten Nukleinsäurefragment 5' von dem komplementären Abschnitt zu dem ersten Nukleinsäurefragment befindet und der komplementäre Abschnitt zu dem dritten Nukleinsäurefragment 5' von dem komplementären Abschnitt zu dem zweiten Nukleinsäurefragment befindet. In einer zweiten möglichen Ausführungsform befindet sich der komplementäre Abschnitt zu dem zweiten Nukleinsäurefragment 3' von dem komplementären Abschnitt zu dem ersten Nukleinsäurefragment und der komplementäre Abschnitt zu dem dritten Nukleinsäurefragment 3' von dem komplementären Abschnitt zu dem zweiten Nukleinsäurefragment.

Wird eine andere Reihenfolge der Marker bzw. Sondensignale und/oder eine Vervielfältigung einzelner Signale nachgewiesen, so wird hierdurch erfindungsgemäß das Vorliegen einer Aberration angezeigt. Die Aberration ist dadurch charakterisiert, dass sie eine oder mehrere Bruchstellen zwischen den komplementären Abschnitten aufweist.

Insbesondere verändert sich die räumlich getrennt voneinander nachweisbare Markerreihenfolge erster - zweiter - dritter Marker
a) bei einer Deletion in und/oder zwischen den drei Abschnitten in die räumlich getrennt voneinander nachweisbare Markerreihenfolge aus ausschließlich dem dritten Marker (d. h. der erste und zweite Marker sind für dieses Chromosom nicht nachweisbar); ausschließlich der Reihenfolge zweiter - dritter Marker (d. h. der erste Marker ist für dieses Chromosom nicht nachweisbar) oder ausschließlich der Reihenfolge erster - dritter Marker (d. h. der zweite Marker ist für dieses Chromosom nicht nachweisbar);
b) bei einer Duplikation in und/oder zwischen den drei Abschnitten in die räumlich getrennt voneinander nachweisbare Markerreihenfolge erster - zweiter - erster - zweiter - dritter Marker; zweiter - erster - erster - zweiter - dritter Marker; oder erster - zweiter - erster - zweiter - dritter Marker; und/oder
c) bei einer Inversion in und/oder zwischen den drei Abschnitten in die räumlich getrennt voneinander nachweisbare Markerreihenfolge zweiter - erster - dritter Marker.

Anders gesagt umfasst die Zusammensetzung in einer Ausführungsform somit drei Sonden, wobei
a) die erste Sonde (Sonde A) ein erstes Nukleinsäurefragment umfasst, das mit einem ersten Marker (Marker A) markiert ist, und
b) die zweite Sonde (Sonde B) ein zweites Nukleinsäurefragment umfasst, das mit einem zweiten Marker (Marker B) markiert ist,
   wobei das erste und das zweite Nukleinsäurefragment jeweils spezifisch an einen komplementären Abschnitt auf einem Chromosom binden und diese komplementären Abschnitte in einer kritischen Region lokalisiert sind, wobei die kritische Region von einer Deletion, Duplikation oder Inversion betroffen sein kann, und die Marker A und B, sofern in und zwischen den Abschnitten keine Aberration vorliegt, Signale A-B bilden (d. h. in der räumlichen Reihenfolge A - B nachgewiesen werden können); und
c) die dritte Sonde (Sonde C) ein drittes Nukleinsäurefragment umfasst, das mit einem dritten Marker markiert ist, wobei das dritte Nukleinsäurefragment spezifisch an einen komplementären Abschnitt auf demselben Chromosom bindet und dieser komplementäre Abschnitt neben der kritischen Region auf den gleichen Chromosomenarm lokalisiert ist, und, sofern in und zwischen dem Abschnitt und der kritischen Region keine Aberration vorliegt, das Signal C bildet; und
somit alle drei Sonden die Signale A-B-C bilden (d. h. in der räumlichen Reihenfolge A - B - C nachgewiesen werden können); wobei
die vorgenannten Signale sich bei einer Deletion zu den Signalen C, oder B-C oder A-C, bei einer Duplikation zu den Signalen A-B-A-B-C, oder B-A-A-B-C oder A-B-B-A-C, und bei einer Inversion zu den Signalen B-A-C verändern.

Die Begriffe "lokalisiert sind" und "lokalisiert ist" werden hierbei derart verstanden, dass Sonde A und Sonde B jeweils in der kritischen Region und analog die Sonde C neben einer kritischen Region, die von einer Deletion oder Inversion betroffen sein kann, hybridisieren bzw. die entsprechenden komplementären Abschnitte in bzw. neben dieser Region angeordnet sind.

Überraschenderweise wurde festgestellt, dass bei dem erfindungsgemäßen Verfahren neue Signalanordnungen B-A-C bei Inversionen, C bei Deletionen, auch B-C oder A-C bei partiellen Deletionen sowie A-B-A-B-C bei Duplikationen, auch B-A-A-B-C oder A-B-B-A-C bei invertierten Duplikationen, entstehen. Das erfindungsgemäße Verfahren erlaubt somit erstmals und überraschend die o. g. Nachweise der o. g. Aberrationen, die mit dem Stand der Technik nicht oder nur sehr unzureichend möglich sind. Dabei ermöglicht eine gleichzeitige Auswertung und Verwendung von drei Markern, z. B. in Form von drei Farben, die erstmalige oder eindeutige Erkennung sowohl einer Deletion oder Duplikation als auch einer Inversion einer gleichen kritischen Region.

Bevorzugt wird die erfindungsgemäße Zusammensetzung und das an anderer Stelle hierin beschriebene erfindungsgemäße Verfahren zum Nachweis, z. B. zur Diagnose, einer der nachfolgenden Inversionen und/oder Deletionen oder Duplikationen eingesetzt (die entsprechenden kritischen genomischen Regionen sind in Klammern angegeben): Angelman-Syndrom (15q11-q13), Cri-du-chat-Syndrom (5p15.2-p15.3), DiGeorge-Syndrom (22q11.2), Miller-Dieker-Syndrom (17p13.3), Prader-Willi-Syndrom (15q11-q13), Shprintzen-Syndrom (22q11.2), Smith-Magenis-Syndrom (17p11.2), Williams-Beuren-Syndrom (7q11.23), Wolf-Hirschhorn-Syndrom (4p16.3), Mikrodeletion 1q21.1 (1q21.1), Mikroduplikation 1 q21.1 (1q21.1), Mikrodeletion 1q41q42 (1q41q42), Mikrodeletion 2p15p16.1 (2p15p16.1), Mikrodeletion 3q29 (3q29), Mikroduplikation 7q11.23 (7q11.23), Mikrodeletion 9q22.3 (9q22.3), Mikrodeletion 12q14 (12q14), Mikrodeletion 14q11.2 (14q11.2), Mikrodeletion 15q13.3 (15q13.3), Mikrodeletion 15q24 (15q24), Mikrodeletion/Duplikation 16p11.2 (16p11.2), Mikrodeletion 16p11.2p12.2, (16p11.2p12.2), Mikrodeletion 16p13.1 (16p13.1), Mikroduplikation 16p13.1 (16p13.1), Potocki-Lupski Syndrom (17p11.2), Mikroduplikation 17p11.2 (17p11.2), Mikrodeletion 17q21.31 (17q21.31), Mikrodeletion 19q13.11 (19q13.11), Distale Mikrodeletion 22q11.2 (22q11.2), Mikroduplikation Xq28 (Xq28), 1p32.1-p31.1 (Mikrodeletionen und -duplikation 1p32-p31), 7q32.2-q34 (Mikrodeletionen 7q33) und 6q22.33-q23.3 (Mikrodeletionen 6q22.33). In einer besonders bevorzugten Ausführungsform wird die erfindungsgemäße Zusammensetzung und das an anderer Stelle hierin beschriebene erfindungsgemäße Verfahren zum Nachweis, z. B. zur Diagnose, einer der nachfolgenden Inversionen und/oder Deletionen oder Duplikationen eingesetzt (die entsprechenden kritischen genomischen Regionen sind in Klammern angegeben): Mikrodeletionen und -duplikation 1 p32-p31 (1p32.1-p31.1), Mikrodeletionen 7q33 (7q32.2-q34) und Mikrodeletionen 6q22.33 (6q22.33-q23.3).

Weiter bevorzugt wird die erfindungsgemäße Zusammensetzung und das an anderer Stelle beschriebene erfindungsgemäße Verfahren zum Nachweis, z. B. zur Diagnose, einer strukturellen Chromosomenaberration eingesetzt, die dadurch charakterisiert ist, dass Zellen einer Parentalgeneration eine Inversion in einer Chromosomenregion aufweisen und Zellen einer aus der Parentalgeneration hervorgegangenen Filialgeneration in der Chromosomenregion eine oder mehrere Deletionen und/oder Duplikationen aufweisen. Es ist dem Fachmann bekannt, dass Zellen einer Filialgeneration durch Zellteilung von Zellen aus der Parentalgeneration oder aus Zellen einer Filialgeneration der Parentalgeneration entstehen. Der Begriff Filialgeneration betrifft in diesem Zusammenhang somit aus der Parentalgeneration entstehende Generationen, z. B. die F₁, F₂, F₃ oder nachfolgende Generationen, vorzugsweise die F₁-Generation.

Erkrankungen, z. B. Tumorerkrankungen, in denen solche Zellen einer Filialgeneration eine Rolle bei der (Tumor-) Krankheitsentstehung oder -progression spielen, sind Fachleuten bekannt und werden exemplarisch z. B. von Hopman et al. (2013, Eur J Cancer, 49(9):2170-8) beschrieben. Die erfindungsgemäße Zusammensetzung und das erfindungsgemäße Verfahren werden vorzugsweise zum Nachweis, z. B. zur Diagnose, einer der vorstehend genannten Erkrankungen eingesetzt, insbesondere der von Hopman *et al.* genannten Erkrankungen.

Insbesondere sind die erfindungsgemäße Zusammensetzung und das erfindungsgemäße Verfahren zum Nachweis, z. B. zur Diagnose, von Inversionen, Deletionen und Duplikationen der gleichen (kritischen) genomischen Region geeignet. Solche Inversionen, Deletionen oder Duplikation der gleichen kritischen genomischen Region können mit Hilfe der Zusammensetzung eindeutig erkannt und nachgewiesen werden.

Die Erfindung betrifft ferner ein Verfahren zum Nachweis, z. B. zur Diagnose, struktureller Chromosomenaberrationen in einer Probe, Schritte umfassend, bei denen man
a) eine biologische Probe, die Interphase-Chromosomen umfasst, mit einer erfindungsgemäßen Zusammensetzung unter Bedingungen in Kontakt bringt, die eine Hybridisierung der Sonden mit komplementären Abschnitten auf den Chromosomen erlauben; und
b) die räumliche Reihenfolge der drei Marker nachweist.

Fachleute auf dem Gebiet der ISH Analysen sind in der Lage, geeignete Bedingungen, die eine Hybridisierung der Sonden mit komplementären Abschnitten auf den Chromosomen erlauben, aufzufinden und zu implementieren. Tests für eine erfolgreiche Hybridisierung der Sonden sind ebenfalls bekannt und Standard auf dem Gebiet der ISH Analysen.

Üblicherweise werden die biologische Probe und die erfindungsgemäße Zusammensetzung miteinander in Kontakt gebracht, indem die Zusammensetzung manuell oder automatisiert auf die Probe aufpipettiert oder die relevanten Probenbereiche durch andere Mittel mit der Zusammensetzung geflutet werden. Entsprechende Verfahren sind Fachleuten bekannt.

In Schritt b) des erfindungsgemäßen Verfahrens wird die räumliche Reihenfolge der drei Marker nachgewiesen. Aus den obigen Ausführungen geht bereits hervor, dass sich die Reihenfolge und auch die Anzahl der an einem Chromosom hybridisierten Sonden nach einem Aberrationsereignis von der Reihenfolge und Anzahl in einem Chromosom unterscheiden kann, das in und zwischen den komplementären Abschnitten nicht von einer Aberration betroffen ist. Somit umfasst der Nachweis der Reihenfolge der drei Marker (in einer Zelle oder an einem Chromosom) auch die Bestimmung der Anzahl der nachweisbaren Markersignale (d. h. die Anzahl des ersten, des zweiten und des dritten Markers) pro Chromosom. Eine Veränderung der Reihenfolge der Marker deutet auf ein Aberrationsereignis hin, z. B. auf eine Inversion. Eine Veränderung der Anzahl der nachweisbaren Marker deutet ebenfalls auf ein Aberrationsereignis hin, eine Verringerung (d. h. der Verlust eines, zweier oder aller drei Marker) deutet z. B. auf eine Deletion hin, eine Vervielfältigung (d. h. eine Duplikation von einem, zwei oder drei Markern, einzeln oder paarweise) deutet auf eine Duplikation hin.

Wie zuvor beschrieben, sind die untersuchten Zellen in der Regel diploid, wobei oft nur eines der Chromosomen von einer Aberration betroffen ist. Ein (in und zwischen den komplementären Abschnitten) nicht-aberrantes Chromosom in der Zelle ist daran zu erkennen, dass die Marker in der räumlichen Reihenfolge (d. h. auch in der nachstehend durch Nennung angegebenen Anzahl) erster - zweiter - dritter Marker nachgewiesen werden.

Das erfindungsgemäße Verfahren ist bevorzugt für den Nachweis von Inversionen und/oder Deletionen oder Duplikationen geeignet bzw. wird für einen solchen Nachweis eingesetzt, vorzugsweise zum Nachweis von Inversionen und/oder Deletionen oder Duplikationen kurzer genomischer Abschnitte. "Kurze genomische Abschnitte" im Sinne der Erfindung sind Abschnitte kleiner als 20 Mb, bevorzugt kleiner als 15 Mb, besonders bevorzugt kleiner als 10 Mb und noch bevorzugter kleiner als 5 Mb. In einer bevorzugten Ausführungsform ist das Verfahren zum Nachweis von Inversionen und/oder Deletionen oder Duplikationen in genomischen Abschnitten, die eine Länge von 5 Mb oder weniger aufweisen, z. B von 0,5 Mb bis 5 Mb.

Das Verfahren wird vorzugsweise mittels Fluoreszenz-in-situ-Hybridisierung (FISH) durchgeführt. In einer bevorzugten Ausführungsform sind die Marker Fluoreszenzfarbstoffe für die Emissionsbereiche Grün, Rot und Gold oder für die Emissionsbereiche Grün, Orange/Rot und Blau.

Bevorzugt wird das erfindungsgemäße Verfahren mittels der FISH-Methode durchgeführt, wobei der erste, zweite und dritte Marker direkt eingebaute (d. h. kovalent mit dem jeweiligen Nukleinsäurefragment verbundene) Fluoreszenzfarbstoffe für die Emissionsbereiche Grün, Orange/Rot bzw. Blau sind. In einer alternativen, bevorzugten Ausführungsform wird das erfindungsgemäße Verfahren mittels der FISH-Methode durchgeführt, wobei der erste, zweite und dritte Marker direkt eingebaute (d. h. kovalent mit dem jeweiligen Nukleinsäurefragment verbundene) Fluoreszenzfarbstoffe für die Emissionsbereiche Grün, Rot und Gold sind.

Der Nachweis in Schritt b) erfolgt vorzugsweise durch die Analyse von Fluoreszenzsignalen (sofern es sich bei den Markern z. B. um Fluoreszenzfarbstoffe handelt). Entsprechende Verfahren sind bekannt. So kann die Analyse z. B. mit Hilfe eines Fluoreszenz-Mikroskops erfolgen.

Alternativ wird das erfindungsgemäße Verfahren mittels der BrISH-Methode durchgeführt, wobei der erste, zweite und dritte Marker Biotin, Digoxigenin bzw. DNP sind. In dieser Ausführungsform kann der Nachweis in Schritt b) z. B. mit Antikörper-gekoppelter alkalischer Phosphatase, Antikörper-gekoppelter Peroxidase und Antikörper-gekoppelter beta-Galactosidase, durchgeführt werden.

### Kurze Beschreibung der Figuren

In den Zeichnungen sind Ausführungsbeispiele der Erfindung schematisch dargestellt. Es zeigen:
- **Fig. 1**: eine schematische Darstellung eines erfindungsgemäßen Verfahrens für den Nachweis einer Deletion, partiellen Deletion, Inversion, Duplikation und/oder invertierten Duplikation einer gleichen kritischen genomischen Region unter Verwendung von drei Sonden;
- **Fig. 2**: FISH-Analysen zum Nachweis von Inversionen und Deletionen der Region 7q32.2-q34 ("MMS 7q33) unter Verwendung der Dreifach-FISH-Sonde "ZytoLight 7q32.2-q34 Triple Probe";
- **Fig. 3**: FISH Analysen zum Nachweis von Deletionen der Region 6q22.33-q23.3 ("MMS 6q22.33") unter Verwendung der Dreifach-FISH-Sonde "ZytoLight 6q22.33-q23.3 Triple Probe"; und
- **Fig. 4:**: FISH-Analysen zum Nachweis von Deletionen der Region 1 p32.1-p31.1 ("MMS 1p32-p31") unter Verwendung der Dreifach-FISH-Sonde "ZytoLight 1 p32.1-p31.1 Probe".

### Ausführlichere Beschreibung der Figuren

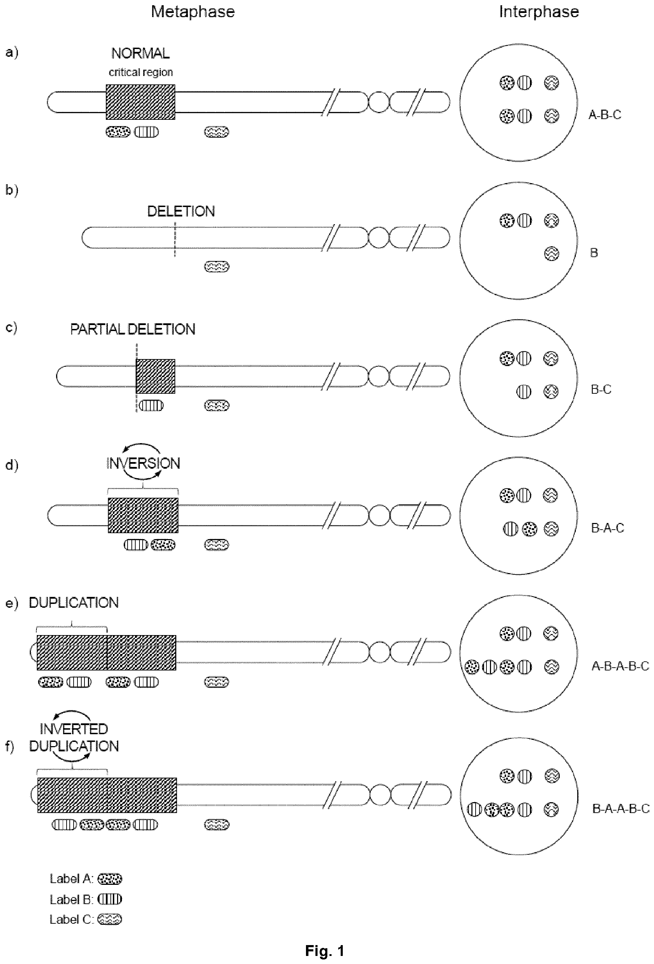

**Fig. 1** zeigt eine schematische Darstellung eines erfindungsgemäßen Verfahrens für den Nachweis einer Deletion, partiellen Deletion, Inversion, Duplikation und/oder invertierten Duplikation einer gleichen kritischen genomischen Region unter Verwendung von drei Markern. Es werden die Signalmuster in der normalen Situation (a), bei einer Deletion (b), bei einer partiellen Deletion (c), bei der Inversion (d), bei einer Duplikation (e) und bei einer invertierten Duplikation (f) gezeigt. Marker A und B einer Dreifach-ISH-Sonde sind in der kritischen Region, die von einer Deletion (auch partiell), Duplikation oder Inversion betroffen sein kann, lokalisiert, und Marker C ist neben der kritischen Region lokalisiert.

In der Interphase einer normalen Zelle (ohne Aberrationen, Fig. 1a) sind drei Signale A, B und C in der Anordnung A-B-C zu erkennen. Bei einer Deletion (Fig. 1b) ist in der Interphase nur ein Signal C sichtbar. Die Interphasezellen, die von einer partiellen Deletion (Fig. 1c) betroffen sind, zeigen nur die Signale B-C (nachweisbar und nicht gezeigt sind auch bei anderen Varianten partieller Deletionen dieser Region die Signale A-C). In einer von einer Inversion betroffenen Interphasenzelle (Fig. 1d) treten die drei Signale A, B und C in einer geänderten Anordnung, nämlich B-A-C, auf. Bei einer Duplikation (Fig. 1e) sind in der Interphase die Signale A-B-A-B-C sichtbar. Die Interphasezellen, die von einer invertierten Duplikation (Fig. 1f) betroffen sind, zeigen die Signale B-A-A-B-C (nachweisbar und nicht gezeigt sind auch bei anderen Varianten invertierter Duplikationen dieser Region die Signale A-B-B-A-C).

Daneben treten bei den zuvor gezeigten Varianten a) bis d) jeweils auch die Signale des anderen, normalen Chromosoms in der Anordnung A-B-C auf (jeweils oben in dem Interphasekern dargestellt).

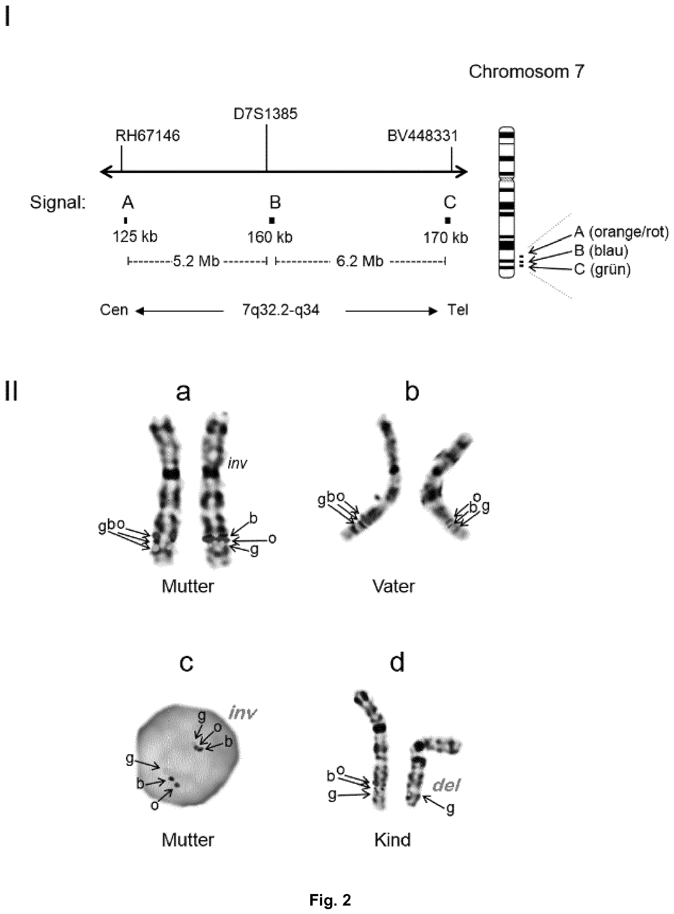

**Fig. 2** zeigt FISH-Analysen zum Nachweis von Inversionen und Deletionen der Region 7q32.2-q34 ("MMS 7q33") unter Verwendung der Dreifach-FISH-Sonde "ZytoLight 7q32.2-q34 Triple Probe".
I: Schematische Darstellung Dreifach-FISH-Sonde "ZytoLight 7q32.2-q34 Triple Probe": Die Sonde besteht aus orange-markierten Polynukleotiden (Marker/Signal A; Absorption bei 547 nm und Emission bei 572 nm; "o"), blau-markierten Polynukleotiden (Marker/Signal B; Absorption bei 426 nm und Emission bei 480 nm; "b") sowie grün-markierten Polynukleotiden (Marker/Signal C; Absorption bei 503 nm und Emission bei 528 nm; "g"), die gegen in der Region 7q32.2-q34 gelegene Sequenzen gerichtet sind. Mit geeigneten Filtersets lässt sich das Signalmuster, welches sich in Interphasen und Metaphasen (z.B. wie hier in invertierter DAPI-Bänderung) normaler Zellen in der Anordnung A-B-C (orange-blau-grün) darstellt, sehr gut sichtbar machen. Die dargestellten Sequenzabschnitte Rh67146, D7S1385 und BV448331 dienen der weiteren Orientierung der Sonde innerhalb der Region.
II: Ergebnisse der FISH-Analysen unter Verwendung von kindlichen, mütterlichen und väterlichen Zellen:
   a) Nachweis einer Inversion mittels Metaphasen-FISH an mütterlichen Zellen: Auf einem Chromosom 7 wechselt die normale Anordnung A-B-C (orange-blau-grün) zu B-A-C (blau-orange-grün, "b-o-g").
   b) Ausschluss von Deletionen, Duplikationen und/oder Inversionen mittels Metaphasen-FISH an väterlichen Zellen: Es zeigt sich auf beiden Chromosomen 7 die Anordnung A-B-C (orange-blau-grün, "o-b-g") einer normalen Zelle.
   c) Nachweis einer Inversion mittels Interphasen-FISH an mütterlichen Zellen: In dem Zellkern wechselt die normale Anordnung A-B-C (orange-blau-grün) zu B-A-C (blau-orange-grün; "b-o-g").
   d) Nachweis einer Deletion mittels Metaphasen-FISH an kindlichen Zellen: Auf einem Chromosom 7 wechselt die normale Anordnung A-B-C (orange-blau-grün) zu dem Auftreten von nur einem Signal C (grün, "g").

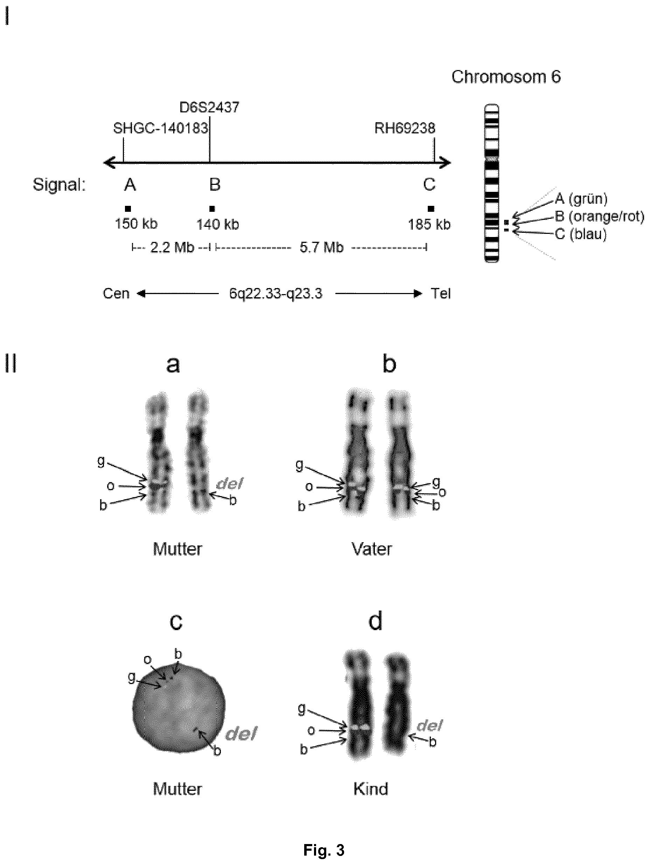

**Fig. 3** zeigt FISH-Analysen zum Nachweis von Deletionen der Region 6q22.33-q23.3 ("MMS 6q22.33") unter Verwendung der Dreifach-FISH-Sonde "ZytoLight 6q22.33-q23.3 Triple Probe".
I: Schematische Darstellung Dreifach-FISH-Sonde "ZytoLight 6q22.33-q23.3 Triple Probe": Die Sonde besteht aus grün-markierten Polynukleotiden (Marker/Signal A; Absorption bei 503 nm und Emission bei 528 nm, "g"), orange-markierten Polynukleotiden (Marker/Signal B; Absorption bei 547 nm und Emission bei 572 nm, "o") sowie blau-markierten Polynukleotiden (Marker/Signal C; Absorption bei 426 nm und Emission bei 480 nm, "b"), die gegen in der Region 6q22.33-q23.3 gelegene Sequenzen gerichtet sind. Mit geeigneten Filtersets lässt sich das Signalmuster, welches sich in Interphasen und Metaphasen (z.B. wie hier in invertierter DAPI-Bänderung) normaler Zellen in der Anordnung A-B-C (grün-orange-blau) darstellt, sehr gut sichtbar machen. Die dargestellten Sequenzabschnitte SHGC-140183, D6S2437 und RH69238 dienen der weiteren Orientierung der Sonde innerhalb der Region.
II: Ergebnisse der FISH-Analysen unter Verwendung von kindlichen, mütterlichen und väterlichen Zellen:
   a) Nachweis einer Deletion mittels Metaphasen-FISH an mütterlichen Zellen: Auf einem Chromosom 6 wechselt die normale Anordnung A-B-C (grün-orange-blau) zu dem Auftreten von nur einem Signal C (blau, "b").
   b) Ausschluss von Deletionen, Duplikationen und/oder Inversionen mittels Metaphasen-FISH an väterlichen Zellen: Es zeigt sich auf beiden Chromosomen 6 die Anordnung A-B-C (grün-orange-blau, "g-o-b") einer normalen Zelle.
   c) Nachweis einer Deletion mittels Interphasen-FISH an mütterlichen Zellen: In dem Zellkern wechselt die normale Anordnung A-B-C (grün-orange-blau) zu dem Auftreten von nur einem Signal C (blau, "b").
   d) Nachweis einer Deletion mittels Metaphasen-FISH an kindlichen Zellen: Auf einem Chromosom 6 wechselt die normale Anordnung A-B-C (grün-orange-blau) zu dem Auftreten von nur einem Signal C (blau, "b").

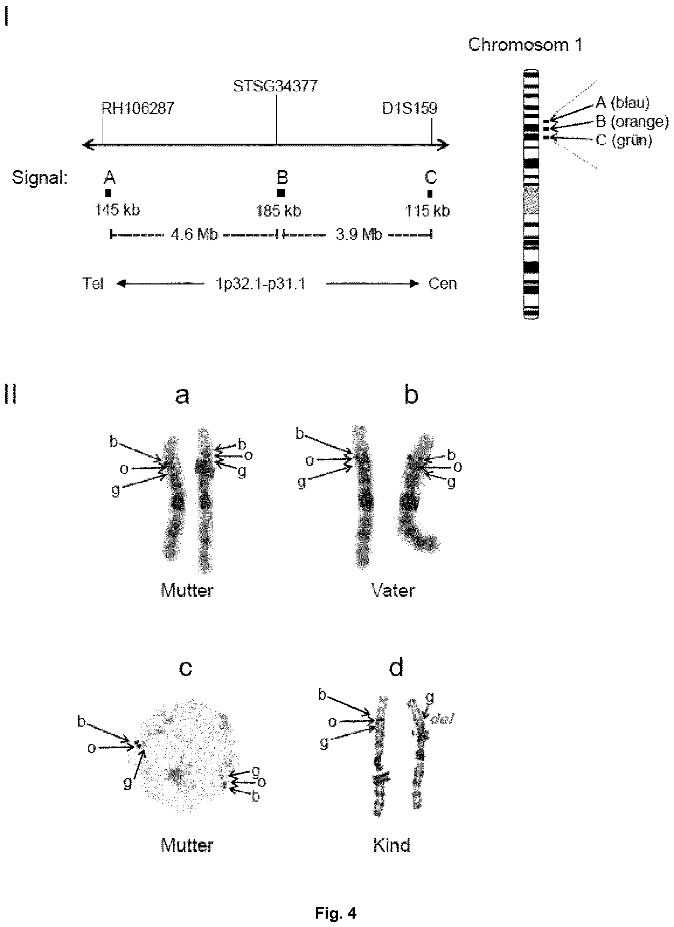

**Fig. 4** zeigt FISH-Analysen zum Nachweis von Deletionen der Region 1p32.1-p31.1 ("MMS 1 p32-p31 ") unter Verwendung der Dreifach-FISH-Sonde "ZytoLight 1 p32.1-p31.1 Probe".
I: Schematische Darstellung Dreifach-FISH-Sonde "ZytoLight 1p32.1-p31.1 Probe": Die Sonde besteht aus blau-markierten Polynukleotiden (Marker/Signal A; Absorption bei 426 nm und Emission bei 480 nm, "b"), orange-markierten Polynukleotiden (Marker/Signal B; Absorption bei 547 nm und Emission bei 572 nm, "o") sowie grün-markierten Polynukleotiden (Marker/Signal C; Absorption bei 503 nm und Emission bei 528 nm, "g"), die gegen in der Region 1 p32.1-p31.1 gelegene Sequenzen gerichtet sind. Mit geeigneten Filtersets lässt sich das Signalmuster, welches sich in Interphasen und Metaphasen (z.B. wie hier in invertierter DAPI-Bänderung) normaler Zellen in der Anordnung A-B-C (blau-orange-grün) darstellt, sehr gut sichtbar machen. Die dargestellten Sequenzabschnitte RH106287, STSG34377 und D1S159 dienen der weiteren Orientierung der Sonde innerhalb der Region.
II: Ergebnisse der FISH-Analysen unter Verwendung von kindlichen, mütterlichen und väterlichen Zellen:
   a) Ausschluss von Deletionen, Duplikationen und/oder Inversionen mittels Metaphasen-FISH an mütterlichen Zellen: Es zeigt sich auf beiden Chromosomen 1 die Anordnung A-B-C (blau-orange-grün, "b-o-g") einer normalen Zelle.
   b) Ausschluss von Deletionen, Duplikationen und/oder Inversionen mittels Metaphasen-FISH an väterlichen Zellen: Es zeigt sich auf beiden Chromosomen 1 die Anordnung A-B-C (blau-orange-grün, "b-o-g") einer normalen Zelle.
   c) Ausschluss von Deletionen, Duplikationen und/oder Inversionen mittels Interphasen-FISH an mütterlichen Zellen: Es zeigt sich in dem Zellkern die Anordnung A-B-C (blau-orange-grün, "b-o-g") einer normalen Zelle.
   d) Nachweis einer Deletion mittels Metaphasen-FISH an kindlichen Zellen: Auf einem Chromosom 1 wechselt die normale Anordnung A-B-C (blau-orange-grün) zu dem Auftreten von nur einem Signal C (grün, "g").

### Weitere Ausführungsformen

In weiteren Ausführungsformen betrifft die Erfindung:
1. Ein Verfahren zum Nachweis mehrerer unterschiedlicher Chromosomen- oder DNS-Bereiche in einer Zelle zum Nachweis struktureller Chromosomenaberrationen, wobei sowohl Inversionen als auch Deletionen oder Duplikation der gleichen kritischen genomischen Region eindeutig erkannt und nachgewiesen werden können, basierend auf direkt oder indirekt markierten Nukleinsäurefragmenten (Sonden), dadurch gekennzeichnet, dass
   - eine mit einem Marker A markierte erste Sonde (Sonde A) und eine mit einem Marker B markierte zweite Sonde (Sonde B) in einer kritischen Region, der von einer Deletion, Duplikation oder Inversion betroffen sein kann, lokalisiert sind und die Signale A-B bilden;
   - eine mit einem Marker C markierte dritte Sonde (Sonde C) neben der kritischen Region auf den gleichen Chromosomenarm lokalisiert ist und das Signal C bildet; und
   - alle drei Sonden die Signale A-B-C bilden, wobei
   sich das vorgenannte Signal A-B-C bei einer Deletion zu dem Signal C oder B-C oder A-C, bei einer Duplikation zu dem Signal A-B-A-B-C oder B-A-A-B-C oder A-B-B-A-C, und bei einer Inversion zu dem Signal B-A-C verändert.
   Eine "Sonde" umfasst, wie an anderer Stelle hierin beschrieben, ein Nukleinsäurefragment, das mit einem Marker markiert ist. Der Marker kann kovalent oder nicht kovalent mit dem Nukleinsäurefragment verbunden sein. Vorzugsweise ist der Marker kovalent mit dem Nukleinsäurefragment verbunden, d. h. "direkt" verbunden.
   "Lokalisiert" bedeutet in diesem Zusammenhang, dass die Sonden mit den betreffenden Chromosomenabschnitten hybridisiert sind.
   Ein Signal wird durch den oder die zugehörigen Marker "gebildet", indem der oder die Marker in der angegebenen räumlichen Reihenfolge nachweisbar sind. Der Nachweis kann z. B. unmittelbar durch Nachweis einer Markerfluoreszenz erfolgen oder durch indirekten Nachweis des Markers mit anderen Molekülen, wie z. B. Antikörpern.
2. Verfahren nach Ausführungsform 1, dadurch gekennzeichnet, dass es sich bei den Sonden um Polynukleotide, modifizierte Polynukleotide oder modifizierte Nukleinsäurefragmente oder Oligonukleotide oder modifizierte Oligonukleotide handelt.
3. Verfahren nach Ausführungsform 2, dadurch gekennzeichnet, dass die Sonden A, B und C unterschiedliche Marker aufweisen.
4. Verfahren nach Ausführungsform 3, dadurch gekennzeichnet, dass der Marker ausgewählt wird aus der Gruppe umfassend Farbstoffe, Farbstoffsubstrate, Chemilumineszenzfarbstoffe (z. B. Acridinium), Radioisotope, Spin-Marker, Enzyme (z. B. Alkalische Phosphatase, Meerrettich-Peroxidase, Sojabohnen-Peroxidase und/oder beta-Galactosidase), Haptene (z. B. Dioxigenin, Biotin, 5(6)-Carboxyfluorescein, Rhodamin, Bromdeoxyuridin, Acetylaminofluoren, Trinitrophenol, Trinitrophenol -Derivat, Estradiol, und/oder DNP), Quantum Dots, Beads, Aminohexyle, Pyrene und Fluoreszenzfarbstoffe (z. B. Fluorescein, Fluorescein-Derivat, 5(6)-Carboxyfluorescein, Coumarin, Coumarin-Derivat, Rhodamin, Rhodamin-Derivat, Tetramethylrhodamin, Lissamin, Texas Red, AMCA, TRITC, IR Farbstoff, Alexa-Farbstoff, Dyomics-Farbstoff, Phycoerythrine, Cascade Blue, Oregon Green 488, Pacific Blue und/oder Rhodamine Green).
5. Verfahren nach Ausführungsform 3, dadurch gekennzeichnet, dass das Verfahren mittels der FISH-Methode unter Verwendung von drei direkt eingebauten (d. h. kovalent mit den Nukleinsäurefragmenten verknüpften) Fluoreszenzfarbstoffen für die Emissionsbereiche Grün, Rot und Blau durchgeführt wird.
6. Verfahren nach Ausführungsform 3, dadurch gekennzeichnet, dass das Verfahren mittels der FISH-Methode unter Verwendung von drei direkt eingebauten Fluoreszenzfarbstoffen für die Emissionsbereiche Grün, Orange/Rot und Gold durchgeführt wird.
7. Verfahren nach Ausführungsform 3, dadurch gekennzeichnet, dass das Verfahren mittels der BrISH-Methode unter Verwendung von Biotin, Digoxigenin und DNP, die sich mit Antikörper-gekoppelter alkalischer Phosphatase, Antikörper-gekoppelter Peroxidase und Antikörper-gekoppelter beta-Galactosidase verbinden, durchgeführt wird.
8. Verfahren nach einer der vorgenannten Ausführungsformen zum Nachweis von Inversionen, Deletionen und/oder Duplikationen.
9. Verfahren nach einer der vorgenannten Ausführungsformen zum Nachweis von Inversionen, Deletionen und/oder Duplikation kurzer genomischer Abschnitte.
10. Verfahren nach Ausführungsform 9, dadurch gekennzeichnet, dass invertierte, deletierte und/oder duplizierte genomische Abschnitte im Bereich kleiner als 20 Mb, bevorzugt kleiner als 15 Mb, besonders bevorzugt kleiner als 10 Mb und noch bevorzugter kleiner als 5 Mb nachgewiesen werden.
11. Verfahren nach einer der vorgenannten Ausführungsformen, dadurch gekennzeichnet, dass in der kritischen genomischen Region der Abstand der beiden Sonden A und B kleiner als 10 Mb, bevorzugt kleiner als 5 Mb, besonders bevorzugt kleiner als 2 Mb und noch bevorzugter kleiner als 1 Mb ist.
12. Verfahren nach einer der vorgenannten Ausführungsformen, dadurch gekennzeichnet, dass der Abstand der Sonde B in der kritischen genomischen Region und der Sonde C neben der kritischen Region zwischen 20 Mb und 5 Mb, bevorzugt zwischen 18 Mb und 6 Mb, besonders bevorzugt zwischen 16 Mb und 7 Mb und noch bevorzugter zwischen 14 Mb und 6 Mb ist.
13. Verfahren nach einer der vorgenannten Ausführungsformen, wobei die nachfolgenden Mikrodeletions- und Mikroduplikationssyndrome und entsprechenden kritischen genomischen Regionen betroffen sind: Angelman-Syndrom (15q11-q13), Cri-du-chat-Syndrom (5p15.2-p15.3), DiGeorge-Syndrom (22q11.2), Miller-Dieker-Syndrom (17p13.3), Prader-Willi-Syndrom (15q11-q13), Shprintzen-Syndrom (22q11.2), Smith-Magenis-Syndrom (17p11.2), Williams-Beuren-Syndrom (7q11.23), Wolf-Hirschhorn-Syndrom (4p16.3), Mikrodeletion 1q21.1 (1q21.1), Mikroduplikation 1q21.1 (1q21.1), Mikrodeletion 1q41q42 (1q41q42), Mikrodeletion 2p15p16.1 (2p15p16.1), Mikrodeletion 3q29 (3q29), Mikroduplikation 7q11.23 (7q11.23), Mikrodeletion 9q22.3 (9q22.3), Mikrodeletion 12q14 (12q14), Mikrodeletion 14q11.2 (14q11.2), Mikrodeletion 15q13.3 (15q13.3), Mikrodeletion 15q24 (15q24), Mikrodeletion/Duplikation 16p11.2 (16p11.2), Mikrodeletion 16p11.2p12.2, (16p11.2p12.2), Mikrodeletion 16p13.1 (16p13.1), Mikroduplikation 16p13.1 (16p13.1), Potocki-Lupski Syndrom (17p11.2), Mikroduplikation 17p11.2 (17p11.2), Mikrodeletion 17q21.31 (17q21.31), Mikrodeletion 19q13.11 (19q13.11), Distale Mikrodeletion 22q11.2 (22q11.2), Mikroduplikation Xq28 (Xq28), 1p32.1-p31.1 (Mikrodeletionen und -duplikation 1p32-p31), 7q32.2-q34 (Mikrodeletionen 7q33) und 6q22.33-q23.3 (Mikrodeletionen 6q22.33).
14. Zusammensetzung zum Nachweis mehrerer unterschiedlicher Chromosomen- oder DNS-Bereiche in einer Zelle zum Nachweis struktureller Chromosomenaberrationen, wobei sowohl Inversionen (einer Parentalgeneration) als auch Deletionen oder Duplikation (einer Filialgeneration) der gleichen kritischen genomischen Region eindeutig erkannt und nachgewiesen werden können, basierend auf direkt oder indirekt markierten Nukleinsäurefragmenten (Sonden), und wobei die Zusammensetzung umfasst
   - eine mit einem Marker A markierte erste Sonde (Sonde A) und eine mit einem Marker B markierte zweite Sonde (Sonde B), die in einer kritischen Region, die von einer Deletion, Duplikation oder Inversion betroffen sein kann, lokalisiert sind und die Signale A-B bilden;
   - eine mit einem Marker C markierten dritte Sonde (Sonde C), die neben der kritischen Region auf den gleichen Chromosomenarm lokalisiert ist und das Signal C bildet; und
   - somit alle drei Sonden die Signale A-B-C bilden, wobei
   sich das vorgenannte Signal A-B-C bei einer Deletion zu dem Signal C oder B-C oder A-C, bei einer Duplikation zu dem Signal A-B-A-B-C oder B-A-A-B-C oder A-B-B-A-C, und bei einer Inversion zu dem Signal B-A-C verändert.
15. Zusammensetzung nach Ausführungsform 14, dadurch gekennzeichnet, dass die Sonden und/oder die Zusammensetzung nach den Ansprüchen 2 - 7 ausgestaltet sind.

### Beispiele

Weitere Vorteile, Kennzeichen und Merkmale der vorliegenden Erfindung werden bei der nachfolgenden detaillierten Beschreibung von Ausführungsbeispielen anhand der beigefügten Figuren deutlich. Allerdings ist die Erfindung nicht auf diese Ausführungsbeispiele beschränkt.

**Beispiel 1:** FISH-Analyse zum Nachweis von Aberrationen der Region 1p32.1-p31.1 ("MMS 1p32-p31") in Blutkulturen verschiedener Probanden unter Verwendung der Triple-FISH-Sonde "ZytoLight 1 p32.1-p31.1 Probe" der Fa. ZytoVision GmbH

Die FISH-Analysen erfolgten an Lymphozyten-Präparaten verschiedener Probanden jeweils aus Vollblut mit angereicherten Metaphasen. Zur Herstellung der Präparate wurde 1 ml Heparin-Blut in 10 ml Lymphozytenmedium mit PHA (Biochrom AG) in einer Zellkulturflasche für 72 Std. bei 37 °C im Wärmeschrank inkubiert. 90 min vor Ende der Inkubationszeit wurden 100 µl Demecolcine pro Kulturflasche zugegeben, um den Zellzyklus in der Metaphase zu arretieren und damit die Anzahl an Metaphasen im Präparat zu erhöhen. Interphase-Zellen sind weiterhin in der Probe nachweisbar. Nach Ende der Inkubation wurden die Zellen in ein Zentrifugenröhrchen überführt und durch Zentrifugation pelletiert. Der Überstand wurde abgenommen und die Zellen in warmer hypotoner KCI-Lösung (Roth) aufgenommen und 10 min bei 37 °C im Wasserbad inkubiert. Die Behandlung in hypotoner Lösung wurde wiederholt bevor tropfenweise frisch angesetztes kaltes Carnoy's Fixativ (Methanol:Eisessig im Verhältnis 3+1, Roth) unter ständigem Schütteln zugegeben wurde. Nach Inkubation für 10 min bei RT und dem Pelletieren der Zellen durch Zentrifugation wurde der Fixierungsschritt noch 3x wiederholt. Danach wurde das Pellet in 1 ml Fixativ resuspendiert und über Nacht bei -20°C fixiert.

Zum Anfertigen der Präparate für die FISH wurde die Lymphozytensuspension abzentrifugiert und das Pellet in einer geeigneten Menge an frischem Fixativ resuspendiert. Anschließend wurden jeweils 1-2 Tropfen der Suspension mittels Pasteur-Pipette in feucht-warmer Umgebung (z.B. in einem Wasserbad) auf entfettete unbeschichtete Objektträger getropft. Nach dem Lufttrocknen wurden die Präparate in einer aufsteigenden Ethanolreihe für je 1 min in 70%, 90% und 96% Ethanol dehydriert. Vor der Verwendung der Präparate wurden diese mind. über Nacht bei -20°C in 70% Ethanol gelagert.

Die Durchführung der FISH begann mit einem Aging-Schritt für 2 min bei 73 °C in 2x SSC-Puffer (aus 20x SSC, ZytoVision GmbH). Direkt daran anschließend erfolgte die proteolytische Vorbehandlung der Präparate durch Auftropfen einer Cytology Pepsin-Lösung (Cytology Pepsin Solution, ZytoVision GmbH) und anschließende Inkubation in einer feuchten Kammer für 10 bei 37 °C. Für die Post-Fixierung erfolgte zunächst eine Inkubation der Präparate für 5 min bei RT in einer Küvette mit 1x TBS (aus 20x TBS, ZytoVision GmbH), gefolgt von einer Inkubation für 5 min in einer Küvette mit gepufferter 1% Formaldehyd-Lösung (Formaldehyde Dilution Buffer Set, ZytoVsion GmbH + 37% Formaldehyd, säurefrei, Roth) und einer weiteren Inkubation für 5 min bei RT in 1x TBS. Zur Dehydrierung der Präparate schloss sich eine aufsteigende Ethanolreihe für je 1 min in 70%, 90% und 96% Ethanol an. Nach Lufttrocknung der Präparate für mind. 30 min wurden jeweils 10 µl der FISH-Sonde ZytoLight 1 p32.1-p31.1 Probe (ZytoVision GmbH) mittels Pipette direkt auf die Präparate aufgetragen. Die Sonde besteht aus blau-markierten Polynukleotiden (Marker/Signal A; Absorption bei 426 nm und Emission bei 480 nm), orange-markierten Polynukleotiden (Marker/Signal B; Absorption bei 547 nm und Emission bei 572 nm) sowie grün-markierten Polynukleotiden (Marker/Signal C; Absorption bei 503 nm und Emission bei 528 nm), die gegen in der Region 1p32.1-p31.1 gelegene Sequenzen gerichtet sind. Die einzelnen Marker A, B und C decken genomische Bereiche einer Größe von 145 kb, 185 kb und 115 kb ab und haben Abstände von 4,6 Mb (Marker A zu B) und 3,9 Mb (Marker B zu C) (vgl. Fig. 4 I). Anschließend wurden 22x22 mm Deckgläser luftblasenfrei aufgelegt und die Kanten mit Fixogum (Marabu) versiegelt. Nach der Denaturierung der Präparate über eine Dauer von 5 min bei 72 °C auf einer Heizplatte wurde die Hybridisierung in einer vorgewärmten feuchten Kammer bei 37 °C über Nacht (ca. 16 Stunden) in einem Wärmeofen durchgeführt. Nach der Hybridisierung wurden Fixogum und Deckgläser entfernt, und es erfolgte die Stringenzwaschung der Präparate für 2 min bei 70 °C in Waschpuffer (Cytology Stringency Wash Buffer SSC, ZytoVision GmbH) in einer Glasküvette gefolgt von einer weiteren Waschung (Cytology Wash Buffer SSC) für 1 min bei RT. Anschließend wurden die Präparate in einer aufsteigenden Ethanolreihe (jeweils 1 min bei RT in 70 %, 90 %, 96 % Ethanol) dehydriert und luftgetrocknet, wobei die Proben vor direktem Lichteinfall geschützt wurden. Nach Applikation der Gegenfärbung (25 µl DAPI DuraTect Solution, ZytoVision GmbH) wurden 24x60 mm Deckgläser luftblasenfrei aufgelegt und die Präparate lichtgeschützt für mindestens 15 min bei RT inkubiert. Anschließend erfolgt die Auswertung am Fluoreszenzmikroskop (Axio Scope.A1 mit Beleuchtungseinheit HXP 120 V, Carl Zeiss Microscopy GmbH) unter Verwendung geeigneter Filtersätze (Dualband Green / Orange-Red Filterset, AHF Analysentechnik; Sp. Aqua HC mFISH Filterset, AHF Analysentechnik); dabei werden auch zur Darstellung eines G-Bandings die DAPI-gefärbten chromosomalen Banden der Metaphasen per Computerprogramm invertiert.

Es zeigten sich bei normalen Probanden ohne chromosomale Aberrationen in der Mehrheit der analysierten Interphase-Kerne und Metaphasen die Signalmuster in der Anordnung A-B-C (blau-orange-grün) (vgl. Fig. 4 II a, b und c).

In Interphase-Zellkernen und Metaphasen von Zellen von Probanden, die von einer Inversion der Region 1p32.1-p31.1 betroffen waren, wechselte die normale Anordnung A-B-C (blau-orange-grün) zu B-A-C (orange blau-grün) (Daten nicht gezeigt).

In Interphase-Zellkernen und Metaphasen von Zellen von Probanden, die von einer Deletion der Region 1 p32.1-p31.1 betroffen waren, wechselte die normale Anordnung A-B-C (blau-orange-grün) zu dem Auftreten von nur einem Signal C (grün) (vgl. Fig. 4 II d).

In Interphase-Zellkernen und Metaphasen von Zellen von Probanden, die von einer Duplikation der Region 1 p32.1-p31.1 betroffen waren, wechselte die normale Anordnung A-B-C (blau-orange-grün) zu A-B-A-B-C (blau-orange-blau-orange-grün) (Daten nicht gezeigt).

**Beispiel 2:** FISH-Analyse zum Nachweis von Aberrationen der Region 7q32.2-q34 ("MMS 7q33") in Blutkulturen verschiedener Probanden unter Verwendung der Triple-FISH-Sonde "ZytoLight 7q32.2-q34 Triple Probe" der Fa. ZytoVision GmbH

Die FISH-Analysen erfolgten an Lymphozyten-Präparaten verschiedener Probanden jeweils aus Vollblut mit angereicherten Metaphasen. Die Herstellung der Präparate sowie Durchführung der FISH (Heparin-Blut bis Auswertung am Fluoreszenzmikroskop) erfolgte wie bei Ausführungsbeispiel 1 beschrieben. In den Präparaten waren sowohl Interphase-Zellkerne als auch Metaphasen nachweisbar.

Abweichend wurden jeweils 10 µl der FISH-Sonde "ZytoLight 7q32.2-q34 Triple Probe" (ZytoVision GmbH) mittels Pipette direkt auf die Präparate aufgetragen. Die Sonde besteht aus orange-markierten Polynukleotiden (Marker/Signal A; Absorption bei 547 nm und Emission bei 572 nm), blau-markierten Polynukleotiden (Marker/Signal B; Absorption bei 426 nm und Emission bei 480 nm) sowie grün-markierten Polynukleotiden (Marker/Signal C; Absorption bei 503 nm und Emission bei 528 nm), die gegen in der Region 7q32.2-q34 gelegene Sequenzen gerichtet sind (Fig. 2 I). Die einzelnen Marker A, B und C decken genomische Bereiche einer Größe von 125 kb, 160 kb und 170 kb ab und haben Abstände von 5,2 Mb (Marker A zu B) und 6,2 Mb (Marker B zu C).

Es zeigten sich bei Probanden mit normalen Zellen ohne chromosomale Aberrationen in der Mehrheit der analysierten Kerne und Metaphasen die Signalmuster in der Anordnung A-B-C (orange-blau-grün) (z. B. Fig. 2 II b).

In Interphase-Zellkernen und Metaphasen von Zellen von Probanden, die von einer Inversion der Region 7q32.2-q34 betroffen waren, wechselte die normale Anordnung A-B-C (orange-blau-grün) zu B-A-C (blau-orange-grün) (Fig. 2 II a und c).

In Zellkernen und Metaphasen von Zellen von Probanden, die von einer Deletion der Region 7q32.2-q34 betroffen waren, wechselte die normale Anordnung A-B-C (orange-blau-grün) zu dem Auftreten von nur einem Signal C (grün) (Fig. 2 II d).

In Zellkernen und Metaphasen von Zellen von Probanden, die von einer partiellen Deletion der Region 7q32.2-q34 betroffen waren, wechselte die normale Anordnung A-B-C (orange-blau-grün) zu B-C (blau-grün) (Daten nicht gezeigt).

**Beispiel 3:** FISH-Analyse zum Nachweis von Aberrationen der Region 6q22.33-q23.3 ("MMS 6q22.33") in Blutkulturen verschiedener Probanden unter Verwendung der Triple-FISH-Sonde "ZytoLight 6q22.33-q23.3 Triple Probe" der Fa. ZytoVision GmbH Die FISH-Analysen erfolgten an Lymphozyten-Präparaten verschiedener Probanden jeweils aus Vollblut mit angereicherten Metaphasen. Die Herstellung der Präparate sowie Durchführung der FISH (Heparin-Blut bis Auswertung am Fluoreszenzmikroskop) erfolgte wie bei Ausführungsbeispiel 1 beschrieben. In den Präparaten waren sowohl Interphase-Zellkerne als auch Metaphasen nachweisbar.

Abweichend wurden jeweils 10 µl der FISH-Sonde "ZytoLight 6q22.33-q23.3 Triple Probe" (ZytoVision GmbH) mittels Pipette direkt auf die Präparate aufgetragen. Die Sonde besteht aus grün-markierten Polynukleotiden (Marker/Signal A; Absorption bei 503 nm und Emission bei 528 nm), orange-markierten Polynukleotiden (Marker/Signal B; Absorption bei 547 nm und Emission bei 572 nm) sowie blau-markierten Polynukleotiden (Marker/Signal C; Absorption bei 426 nm und Emission bei 480 nm), die gegen in der Region 6q22.33-q23.3 gelegene Sequenzen gerichtet sind (Fig. 3 I). Die einzelnen Marker A, B und C decken genomische Bereiche einer Größe von 150 kb, 140 kb und 185 kb ab und haben Abstände von 2,2 Mb (Marker A zu B) und 5,7 Mb (Marker B zu C).

Es zeigten sich bei Probanden mit normalen Zellen ohne chromosomale Aberrationen in der Mehrheit der analysierten Kerne und Metaphasen die Signalmuster in der Anordnung A-B-C (grün-orange-blau) (Fig. 3 II b).

In Interphase-Zellkernen und Metaphasen von Zellen von Probanden, die von einer Inversion der Region 6q22.33-q23.3 betroffen waren, wechselte die normale Anordnung A-B-C (grün-orange-blau) zu B-A-C (orange-grün-blau) (Daten nicht gezeigt).

In Interphase-Zellkernen und Metaphasen von Zellen von Probanden, die von einer Deletion der Region 6q22.33-q23.3 betroffen waren, wechselte die normale Anordnung A-B-C (grün-orange-blau) zu dem Auftreten von nur einem Signal C (blau) (Fig. 3 II a, c und d).

In Interphase-Zellkernen und Metaphasen von Zellen von Probanden, die von einer partiellen Deletion der Region 6q22.33-q23.3 betroffen waren, wechselte die normale Anordnung A-B-C (grün-orange-blau) zu B-C (orange-blau) (Daten nicht gezeigt).

Obwohl die vorliegende Erfindung anhand der Ausführungsbeispiele detailliert beschrieben worden ist, ist für den Fachmann selbstverständlich, dass die Erfindung nicht auf diese Ausführungsbeispiele beschränkt ist, sondern dass vielmehr Abwandlungen in der Weise möglich sind, dass einzelne Merkmale weggelassen oder andersartige Kombinationen der vorgestellten Einzelmerkmale verwirklicht werden können, sofern der Schutzbereich der beigefügten Ansprüche nicht verlassen wird. Die vorliegende Offenbarung schließt sämtliche Kombinationen der vorgestellten Einzelmerkmale ein. *****

## Patentansprüche

1. Zusammensetzung zum Nachweis struktureller Chromosomenaberrationen in einer Probe, umfassend drei Sonden, wobei
a) die erste Sonde ein erstes Nukleinsäurefragment umfasst, das mit einem ersten Marker markiert ist,
b) die zweite Sonde ein zweites Nukleinsäurefragment umfasst, das mit einem zweiten Marker markiert ist, und
c) die dritte Sonde ein drittes Nukleinsäurefragment umfasst, das mit einem dritten Marker markiert ist,
wobei der erste, der zweite und der dritte Marker verschiedene Marker sind,
wobei das erste, das zweite und das dritte Nukleinsäurefragment jeweils spezifisch an einen komplementären Abschnitt auf einem Chromosom binden und die drei Abschnitte auf demselben Chromosom liegen, und
wobei die drei Sonden im spezifisch an das Chromosom gebundenen Zustand Abstände voneinander aufweisen, durch welche die drei Marker räumlich getrennt voneinander nachgewiesen werden können und, sofern in und zwischen den Abschnitten keine Aberration vorliegt, in der räumlichen Reihenfolge erster - zweiter - dritter Marker nachgewiesen werden können.

2. Zusammensetzung nach Anspruch 1, wobei im spezifisch gebundenen Zustand der Abstand zwischen dem ersten Nukleinsäurefragment und dem zweiten Nukleinsäurefragment und der Abstand zwischen dem zweiten Nukleinsäurefragment und dem dritten Nukleinsäurefragment jeweils weniger als 20 Mb ist.

3. Zusammensetzung nach einem der vorangehenden Ansprüche, wobei im spezifisch gebundenen Zustand der Abstand zwischen dem ersten Nukleinsäurefragment und dem zweiten Nukleinsäurefragment weniger als 10 Mb ist.

4. Zusammensetzung nach einem der vorangehenden Ansprüche, wobei im spezifisch gebundenen Zustand der Abstand zwischen dem ersten Nukleinsäurefragment und dem zweiten Nukleinsäurefragment und der Abstand zwischen dem zweiten Nukleinsäurefragment und dem dritten Nukleinsäurefragment jeweils mindestens 1 Mb ist.

5. Zusammensetzung nach einem der vorangehenden Ansprüche, wobei sich die räumlich getrennt voneinander nachweisbare Markerreihenfolge erster - zweiter - dritter Marker
a) bei einer Deletion in und/oder zwischen den drei Abschnitten in die räumlich getrennt voneinander nachweisbare Markerreihenfolge aus ausschließlich dem dritten Marker; ausschließlich der Reihenfolge zweiterdritter Marker oder ausschließlich der Reihenfolge erster - dritter Marker verändert;
b) bei einer Duplikation in und/oder zwischen den drei Abschnitten in die räumlich getrennt voneinander nachweisbare Markerreihenfolge erster - zweiter - erster - zweiter - dritter Marker; zweiter - erster - erster - zweiter - dritter Marker; oder erster - zweiter - erster - zweiter - dritter Marker verändert; und/oder
c) bei einer Inversion in und/oder zwischen den drei Abschnitten in die räumlich getrennt voneinander nachweisbare Markerreihenfolge zweiter - erster - dritter Marker verändert.

6. Zusammensetzung nach einem der vorangehenden Ansprüche, wobei die Sonden geeignet sind, eine Inversion, Deletion oder Duplikation eines genomischen Abschnitts nachzuweisen, die weniger als 20 Mb umfasst.

7. Zusammensetzung nach einem der vorangehenden Ansprüche, wobei das erste und zweite Nukleinsäurefragment beide in demselben Chromosomenbereich binden, wobei der Chromosomenbereich ausgewählt ist aus der Gruppe bestehend aus 15q11-q13 (Angelman-Syndrom), 5p15.2-p15.3 (Cri-du-chat-Syndrom), 22q11.2 (DiGeorge-Syndrom), 17p13.3 (Miller-Dieker-Syndrom), 15q11-q13 (Prader-Willi-Syndrom), 22q11.2 (Shprintzen-Syndrom), 17p11.2 (Smith-Magenis-Syndrom), 7q11.23 (Williams-Beuren-Syndrom), 4p16.3 (Wolf-Hirschhorn-Syndrom), 1q21.1 (Mikrodeletion 1q21.1), 1q21.1 (Mikroduplikation 1q21.1), 1q41q42 (Mikrodeletion 1q41q42), 2p15p16.1 (Mikrodeletion 2p15p16.1), 3q29 (Mikrodeletion 3q29), 7q11.23 (Mikroduplikation 7q11.23), 9q22.3 (Mikrodeletion 9q22.3), 12q14 (Mikrodeletion 12q14), 14q11.2 (Mikrodeletion 14q11.2), 15q13.3 (Mikrodeletion 15q13.3), 15q24 (Mikrodeletion 15q24), 16p11.2 (Mikrodeletion/Duplikation 16p11.2), 16p11.2p12.2 (Mikrodeletion 16p11.2p12.2), 16p13.1 (Mikrodeletion 16p13.1), 16p13.1 (Mikroduplikation 16p13.1), 17p11.2 (Potocki-Lupski Syndrom), 17p11.2 (Mikroduplikation 17p11.2), 17q21.31 (Mikrodeletion 17q21.31), 19q13.11 (Mikrodeletion 19q13.11), 22q11.2 (Distale Mikrodeletion 22q11.2), Xq28 (Mikroduplikation Xq28), 1 p32.1-p31.1 (Mikrodeletionen und -duplikation 1p32-p31), 7q32.2-q34 (Mikrodeletionen 7q33) und 6q22.33-q23.3 (Mikrodeletionen 6q22.33).

8. Zusammensetzung nach einem der vorangehenden Ansprüche, wobei die Marker ausgewählt sind aus der Gruppe bestehend aus Farbstoffen, wie Fluoreszenzfarbstoffen und Chemilumineszenzfarbstoffen; Farbstoffsubstraten; Radioisotopen, Spin-Labeln, Enzymen, Haptenen, Quantum Dots, Beads, Aminohexylen und Pyren.

9. Zusammensetzung nach einem der vorangehenden Ansprüche, wobei die Zusammensetzung für den Nachweis von Mikrodeletionen, Mikroduplikationen und/oder Mikroinversionen geeignet ist.

10. Zusammensetzung nach einem der vorangehenden Ansprüche, wobei der erste, zweite und dritte Marker Fluoreszenzfarbstoffe und jeweils kovalent mit dem ersten, zweiten bzw. dritten Nukleinsäurefragment verbunden sind.

11. Zusammensetzung nach einem der vorangehenden Ansprüche, wobei die Nukleinsäurefragmente eine Länge von 100 kb bis 300 kb aufweisen.

12. Zusammensetzung nach einem der vorangehenden Ansprüche, wobei die komplementären Abschnitte zu dem ersten und dem zweiten Nukleinsäurefragment in einem Chromosomenabschnitt sind, von dem bekannt ist, dass es zu einem oder mehreren Aberrationsereignissen in dem Abschnitt kommen kann, und wobei der komplementäre Abschnitt zu dem dritten Nukleinsäurefragment außerhalb dieses Chromosomenabschnitts ist, und wobei eine bekannte potentielle Bruchstelle zwischen den zu dem zweiten und dem dritten Nukleinsäurefragment komplementären Abschnitten liegt.

13. Verfahren zum Nachweis struktureller Chromosomenaberrationen in einer Probe, Schritte umfassend, bei denen man
a) eine biologische Probe, die Chromosomen umfasst, mit einer Zusammensetzung nach einem der vorangehenden Ansprüche unter Bedingungen in Kontakt bringt, die eine Hybridisierung der Sonden mit komplementären Abschnitten auf den Chromosomen erlauben; und
b) die räumliche Reihenfolge der drei Marker nachweist.

14. Verfahren nach Anspruch 13, wobei das Verfahren mittels Fluoreszenz-in-situ-Hybridisierung (FISH) durchgeführt wird und wobei die Marker Fluoreszenzfarbstoffe für die Emissionsbereiche Grün, Orange/Rot und Gold oder für die Emissionsbereiche Grün, Rot und Blau sind.

15. Verfahren nach einem der Ansprüche 13 bis 14, wobei das Verfahren mittels Hellfeld-in-situ-Hybridisierung (BrISH) durchgeführt wird.
